(19) Europäisches Patentamt
European Patent Office
Office européen des brevets

(11) Veröffentlichungsnummer: **0 117 474**
**B1**

(12) **EUROPÄISCHE PATENTSCHRIFT**

(45) Veröffentlichungstag der Patentschrift: **29.10.86**

(51) Int. Cl.⁴: **C 07 D 215/56**, C 07 D 405/12, A 61 K 31/495

(21) Anmeldenummer: **84101443.4**

(22) Anmeldetag: **13.02.84**

(54) **Chinoloncarbonsäuren, Verfahren zu ihrer Herstellung sowie diese enthaltende antibakterielle Mittel.**

(30) Priorität: **25.02.83 DE 3306772**

(43) Veröffentlichungstag der Anmeldung:
**05.09.84 Patentblatt 84/36**

(45) Bekanntmachung des Hinweises auf die Patenterteilung:
**29.10.86 Patentblatt 86/44**

(84) Benannte Vertragsstaaten:
**AT BE CH DE FR GB IT LI LU NL SE**

(56) Entgegenhaltungen:
**EP - A - 0 004 279**
**EP - A - 0 049 355**
**EP - A - 0 078 362**
**EP - A - 0 090 424**
**DE - A - 2 840 910**

**CHEMICAL ABSTRACTS, Band 93, Nr. 21, 24. November 1980, Columbus, Ohio,USA: KOGA, HIROSHI "Structure-activity relationships of antibacterial 6,7-and 7,8-disubstituted 1-alkyl-1,4-dihydro-4-oxoquinoline-3-carboxylic acids" Seite 128, Spalte 1, Auszug Nr. 198 382n**
**CHEMICAL ABSTRACTS, Band 93, Nr. 23, 8. Dezember 1980, Columbus, Ohio,USA: KYORIN PHARMACEUTICAL CO. "Substituted**

(73) Patentinhaber: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**
Erfinder: **Zeiler, Hans-Joachim, Dr., Elsbeekerstrasse 46, D-5620 Velbert 15 (DE)**
Erfinder: **Metzger, Karl Georg, Dr., Pahlkestrasse 75, D-5600 Wuppertal (DE)**

(56) Entgegenhaltungen: (Fortsetzung)
**quinolinecarboxylic acid derivatives" Seite 535, Spalte 1, Auszug Nr. 220 772p**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft Chinoloncarbonsäuren, Verfahren zu ihrer Herstellung sowie diese enthaltende antibakterielle Mittel.

In der EP-A 49 355, DE-A 2 840 910 und in Chemical Abstracts, Bd. 93, 1980, 220772p, Seite 535 werden Chinoloncarbonsäuren mit antibakterieller Wirkung beschrieben.

Es wurde gefunden, dass die neuen Chinoloncarbonsäuren der Formel (I)

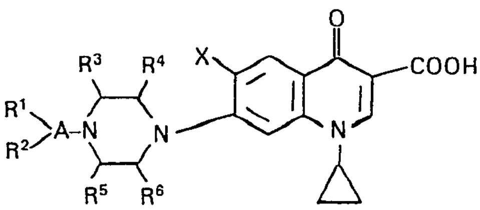

(I)

in der

A für geradkettiges oder verzweigtes Alkylen mit 1 bis 6 Kohlenstoffatomen oder einen Rest >C=CH–,

$R^1$ für Alkoxycarbonyl mit 1 bis 6 Kohlenstoffatomen im Alkylteil, Benzyloxycarbonyl, Carboxy, gegebenenfalls durch 1 oder 2 Methyl- oder Ethylreste substituiertes Carbamoyl, Cyano, Dialkoxyphosphonyl oder Alkylsulfonyl mit 1 bis 4 Kohlenstoffatomen im Alkylteil,

$R^2$ für Wasserstoff, Alkoxycarbonyl mit 1 bis 6 Kohlenstoffatomen im Alkylteil, Benzyloxycarbonyl, Carbamoyl, Cyano, Chlor, Acetyl, Alkyl mit 1 bis 3 Kohlenstoffatomen oder Phenyl steht, wobei $R^1$ und $R^2$ auch gemeinsam mit dem Kohlenstoffatom, das sie substituieren, einen 2-Oxotetrahydrofurylring bilden können und

$R^3$, $R^4$, $R^5$ und $R^6$ gleich oder verschieden sein können und für Wasserstoff, Methyl, Ethyl, n- oder i-Propyl stehen, und

X für Wasserstoff, Halogen, vorzugsweise Fluor oder Chlor, oder Nitro steht, und deren pharmazeutisch verwendbare Säureadditions-, Alkali- und Erdalkalisalze und Hydrate eine gute antibakterielle Wirkung sowohl gegen grampositive als auch gramnegative Bakterien aufweisen.

Bevorzugt sind solche Verbindungen der Formel (I), in denen die Symbole folgende Bedeutungen haben:

A = geradkettiges oder verzweigtes Alkylen mit 1 bis 5 C-Atomen oder >C=CH–,

$R^1$ = Alkoxycarbonyl mit 1 bis 5 C-Atomen im Alkylteil, Benzyloxycarbonyl, Carboxy, gegebenenfalls durch 1 oder 2 Methyl- oder Ethylreste substituiertes Carbamoyl, Cyano, Methyl- oder Ethylsulfonyl,

$R^2$ = Wasserstoff, Alkoxycarbonyl mit 1 bis 5 C-Atomen im Alkylteil, Benzyloxycarbonyl, Carbamoyl, Cyano, Chlor, Cetyl, Alkyl mit 1 bis 2 Kohlenstoffatomen, Phenyl, wobei $R^1$ und $R^2$ auch gemeinsam mit dem C-Atom, das sie substituieren, einen 2-Oxo-tetrahydrofurylring bilden können,

$R^3$, $R^4$, $R^5$ und $R^6$ = Wasserstoff, Methyl oder Ethyl,

X = Wasserstoff, Fluor, Chlor oder Nitro.

Besonders bevorzugt sind solche Verbindungen der Formel (I), in denen die Symbole folgende Bedeutungen haben:

A = geradkettiges Alkylen mit 1 bis 5 C-Atomen oder >C=CH–,

$R^1$ = Alkoxycarbonyl mit 1 bis 4 C-Atomen im Alkylteil, Benzyloxycarbonyl, Carboxy, Carbamoyl, Cyano, Methylsulfonyl,

$R^2$ = Wasserstoff, Alkoxycarbonyl mit 1 bis 3 C-Atomen im Alkylteil, Cyano, Chlor, Acetyl, Phenyl, wobei $R^1$ und $R^2$ auch gemeinsam mit dem C-Atom, das sie substituieren, einen 2-Oxo-tetrahydro-3-furylring bilden können,

$R^3$ = Wasserstoff, Methyl, Ethyl,

$R^4$ = Wasserstoff,

$R^5$ = Wasserstoff, Methyl,

$R^6$ = Wasserstoff,

X = Wasserstoff, Fluor, Chlor, Nitro.

Weiterhin wurde gefunden, dass man die erfindungsgemässen Verbindungen der Formel (I) erhält, wenn man eine Verbindung der Formel (II)

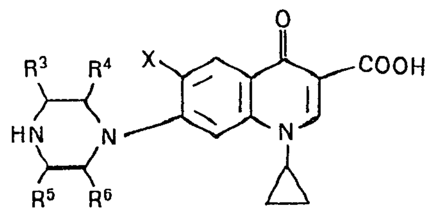

(II)

in welcher

X, $R^3$, $R^4$, $R^5$ und $R^6$ die oben angegebene Bedeutung haben, mit einer Verbindung der Formel (III)

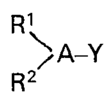

(III)

in welcher

$R^1$, $R^2$ und A die oben angegebene Bedeutung haben und

Y für Halogen, vorzugsweise Chlor, Brom oder Jod, $CH_3O-SO_2-O$, $C_2H_5O-SO_2-O$, Methoxy oder Ethoxy steht, umsetzt (Methode A).

Man erhält erfindungsgemässe Verbindungen der Formel (I) auch, wenn man Verbindungen der Formel (II)

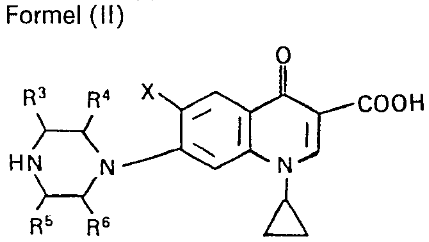

(II)

mit Verbindungen der Formel (IV)

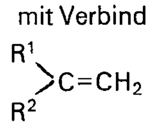

(IV)

in welcher

$R^1$ und $R^2$ die oben angegebene Bedeutung haben,

umsetzt, wobei erfindungsgemässe Verbindungen der Formel (Ia) (I; A = >CH–CH$_2$–)

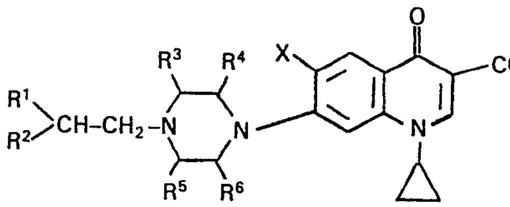

(Ia)

entstehen (Methode B).

Man erhält erfindungsgemässe Verbindungen der Formel (I) auch, wenn man Verbindungen der Formel (V)

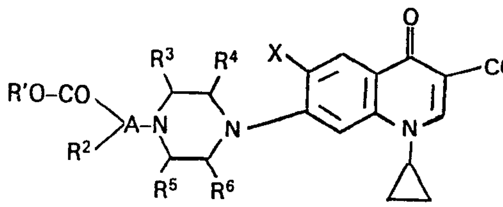

(V)

in welcher

$R^2$, $R^3$, $R^4$, $R^5$, $R^6$ und X die oben angegebene Bedeutung haben und

R' für Alkyl mit 1 bis 6 Kohlenstoffatomen oder Benzyl steht, unter alkalischen oder sauren Bedingungen oder im Falle von R' = Benzyl auch unter hydrogenolytischen Bedingungen umsetzt, wobei erfindungsgemässe Verbindungen der Formel (Ib) = (I, $R^1$ = COOH) entstehen (Methode C).

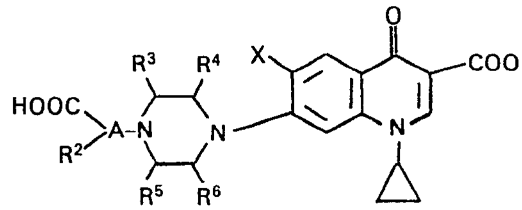

(Ib)

Überraschenderweise zeigen die erfindungsgemässen Chinoloncarbonsäuren eine erheblich höhere antibakterielle Wirkung als die bekannte 1-Ethyl-6-fluor-1,4-dihydro- 4-oxo-7-(1-piperazinyl)- 3-chinolincarbonsäure (Norfloxacin). Die erfindungsgemässen Stoffe stellen somit eine Bereicherung der Pharmazie dar.

Verwendet man beispielsweise bei der Umsetzung von (II) mit (III) nach Methode A 1-Cyclopropyl-6-fluor-1,4-dihydro-4-oxo-7- (1-piperazinyl)-3-chinolincarbonsäure und Bromessigsäureethylester als Ausgangsverbindungen, so kann der Reaktionsablauf durch das folgende Formelschema wiedergegeben werden:

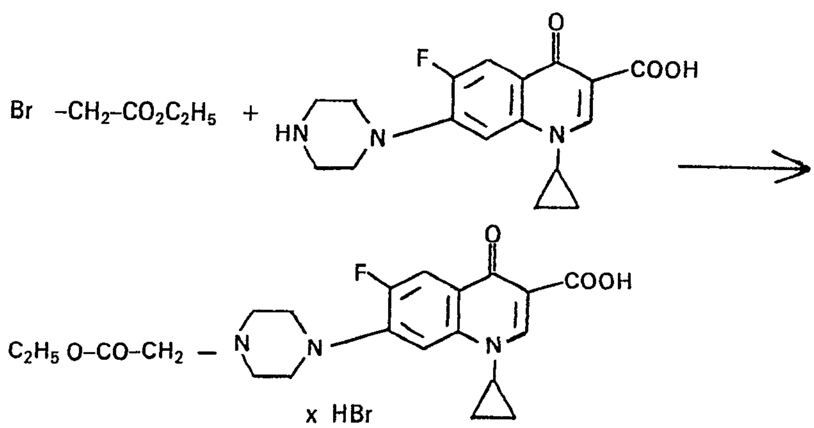

Verwendet man beispielsweise bei der Umsetzung von (II) mit (IV) nach Methode B 1-Cyclopropyl-6-fluor-1,4-dihydro- 4-oxo-7-(1-piperazinyl)- 3-chinolincarbonsäure und Acrylnitril als Ausgangssubstanzen, so kann der Reaktionsablauf durch das folgende Formelschema wiedergegeben werden

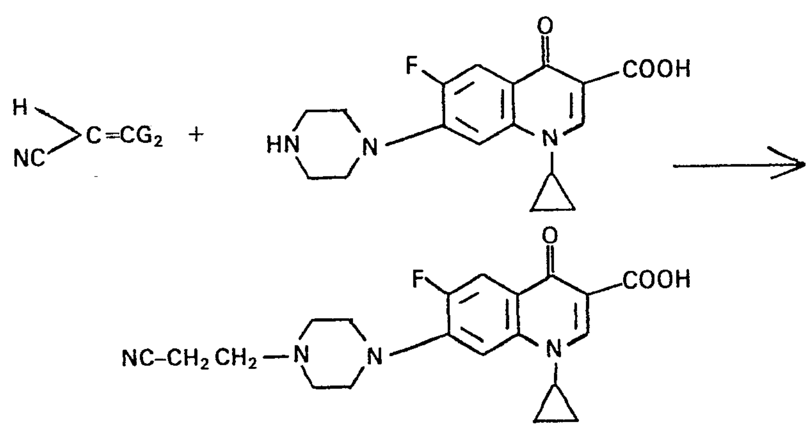

Verwendet man beispielsweise bei der Verseifung von (V) nach Methode C 1-Cyclopropyl-7-(4-ethoxy- carbonylmethyl- 1-piperazinyl)-6-fluor-1,4-dihydro-4-oxo-3-chinolincarbonsäure und Schwefelsäure als Ausgangsverbindungen, so kann der Reaktionsablauf durch das folgende Formelschema wiedergegeben werden:

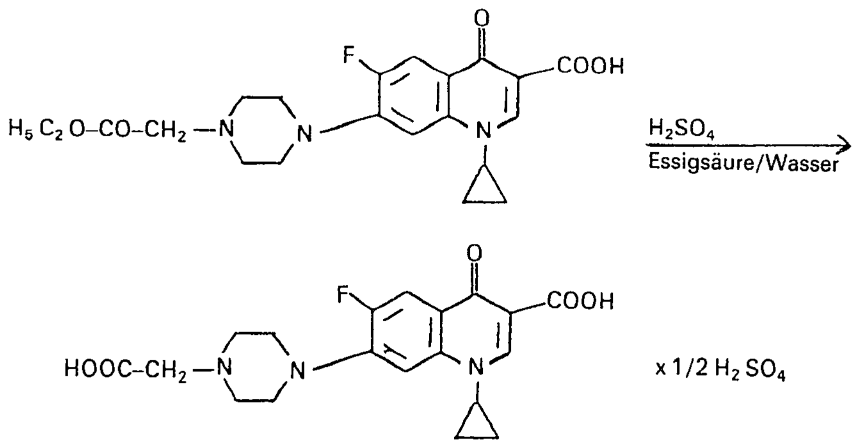


Die als Ausgangsverbindungen verwendeten Verbindungen der Formel (II) können durch Umsetzung von Verbindungen der Formel (VI)

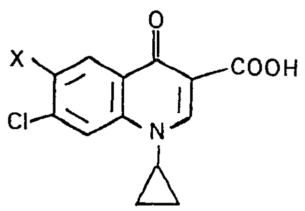

(VI)

mit Piperazin oder Piperazinderivaten der Formel (VII)

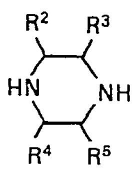

(VII)

hergestellt werden. Man arbeitet hierbei in einem Verdünnungsmittel wie Dimethylsulfoxid, Hexamethylphosphorsäuretrisamid, Sulfolan, Wasser, einem Alkohol oder Pyridin bei Temperaturen von 20° bis 200 °C, vorzugsweise bei 80° bis 180 °C. Bei der Durchführung des Verfahrens setzt man auf 1 Mol Carbonsäure VI 1 bis 15 Mol der Verbindung VII ein. Bei Verwendung äquivalenter Mengen der Carbonsäure VI und des Piperazinderivates VII führt man die Umsetzung in Gegenwart eines säurebindenden Mittels, beispielsweise Triethylamin, 1,4-Diaza-bicyclo- [2,2,2]octan oder 1,8-Diaza-bicyclo[5,4,0]undec-7-en durch.

Als Beispiele für die auf diese Weise herstellbaren Ausgangsprodukte der Formel (II) seien genannt:

1-Cyclopropyl -6- fluor -1,4- dihydro -4- oxo -7-(1-piperazinyl) -3- chinolincarbonsäure,
1-Cyclopropyl -6- fluor -1,4-dihydro-4-oxo-7-(2,4-dimethyl-1-piperazinyl)-3-chinolincarbonsäure,
1-Cyclopropyl-6-fluor-1,4-dihydro-4-oxo-7-(3,5-dimethyl-1-piperazinyl)-3-chinolincarbonsäure,
1-Cyclopropyl-6-fluor-1,4-dihydro-4-oxo-7-(3-methyl-1-piperazinyl)-3-chinolincarbonsäure,
1-Cyclopropyl-6-fluor-1,4-dihydro-4-oxo-7-(3-ethyl-1-piperazinyl)-3-chinolincarbonsäure,
1-Cyclopropyl-6- fluor-1,4-dihydro- 4-oxo-7-(3,5-diethyl-1- piperazinyl)- 3-chinolincarbonsäure,
1-Cyclopropyl- 6-fluor- 1,4- dihydro- 4-oxo-7-(2,3,5-trimethyl- 1-piperazinyl)- 3- chinolincarbonsäure,
1-Cyclopropyl- 6-fluor- 1,4- dihydro- 4-oxo-7-(2,3,5,6- tetramethyl- 1-piperazinyl)- 3-chinolincarbonsäure,
1-Cyclopropyl- 1,4-dihydro- 4-oxo- 7- (1-piperazinyl)- 3- chinolincarbonsäure,
1-Cyclopropyl- 1,4-dihydro- 6-nitro- 4-oxo-7-(1-piperazinyl)- 3-chinolincarbonsäure,
6-Chlor- 1-cyclopropyl- 1,4- dihydro- 4-oxo-7-(1- piperazinyl)- 3-chinol incarbonsäure.

Die als Zwischenprodukt verwendete 7-Chlor-1-cyclopropyl- 6-fluor- 1,4- dihydro- 4-oxo- 3-chinolincarbonsäure der Formel VIa (VI; X = F) kann gemäss folgendem Reaktionsschema hergestellt werden:

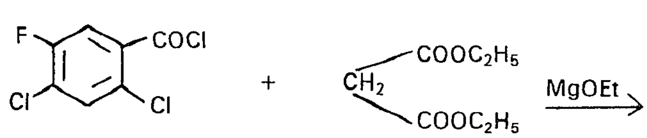


(1) (2)

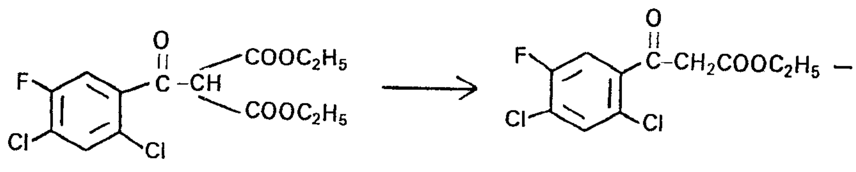

(3) (4)

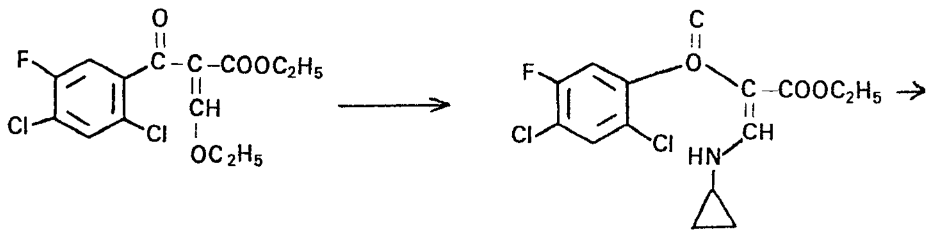

(5) (6)

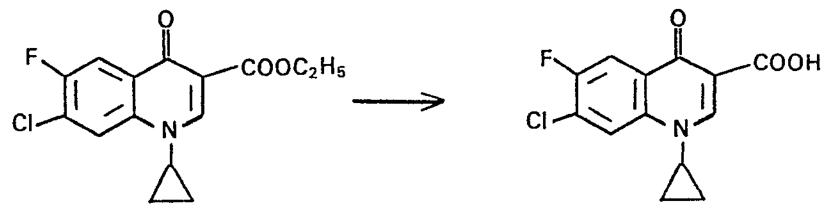

(7) (VIa)

Danach wird Malonsäurediethylester (2) in Gegenwart von Magnesiumalkoholat mit 2,4-Dichlor- 5-fluor- benzoylchlorid (1) (Deutsche Patentanmeldung Nr. 3 142 856.8) zum Acylmalonester (3) acyliert (Organikum, 3. Auflage, 1964, S. 438).

Durch partielle Verseifung und Decarboxylierung von (3) in wässrigem Medium mit katalytischen Mengen p-Toluolsulfonsäure erhält man in guter Ausbeute den Aroylessigsäureethylester (4), der mit o-Ameisensäuretriethylester/Acetanhydrid in den 2-(2,4-Dichlor-5-fluor-benzoyl)-3-ethoxy-axrylsäureethylester (5) übergeht. Die Umsetzung von (5) mit Cyclopropylamin in einem Lösungsmittel, wie z.B. Methylenchlorid, Alkohol, Chloroform, Cyclohexan oder Toluol, führt in leicht exothermer Reaktion zum gewünschten Zwischenprodukt (6).

Die Cyclisierungsreaktion (6) → (7) wird in einem Temperaturbereich von etwa 60° bis 280 °C, bevorzugt 80° bis 180 °C durchgeführt.

Als Verdünnungsmittel können Dioxan, Dimethylsulfoxid, N-Methyl-pyrrolidon, Sulfolan, Hexamethylphosphorsäuretriamid und bevorzugt N,N-Dimethylformamid verwendet werden.

Als Säurebinder kommen für diese Reaktionsstufe Kaliumtert.-butanolat, Butyl-lithium, Lithium-phenyl, Phenyl-magnesiumbromid, Natriummethylat, Natriumhydrid und besonders bevorzugt Kalium- oder Natriumcarbonat in Betracht. Es kann vorteilhaft sein, einen Überschuss von 10 Mol-% an Base einzusetzen.

Die in dem letzten Schritt erfolgende Esterhydrolyse von (7) unter basischen oder sauren Bedingungen führt zur 7-Chlor-1-cyclopropyl-6-fluor- 1,4-dihydro- 4-oxo- 3-chinolincarbonsäure VIa.

Das als Ausgangsmaterial für diesen Syntheseweg verwendete 2,4-Dichlor-5-fluor-benzoylchlorid (1) und die entsprechende Carbonsäure sowie das für die Herstellung von (1) benötigte 3-Fluor-4,6-dichlortoluol (10) werden gemäss nachstehendem Formelschema, ausgehend von 2,4-dichlor-5-methyl-anilin (8), hergestellt:

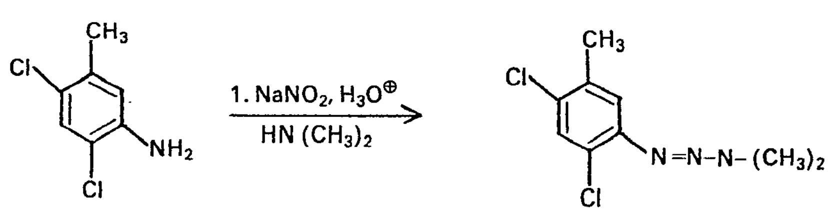


(8) (9)

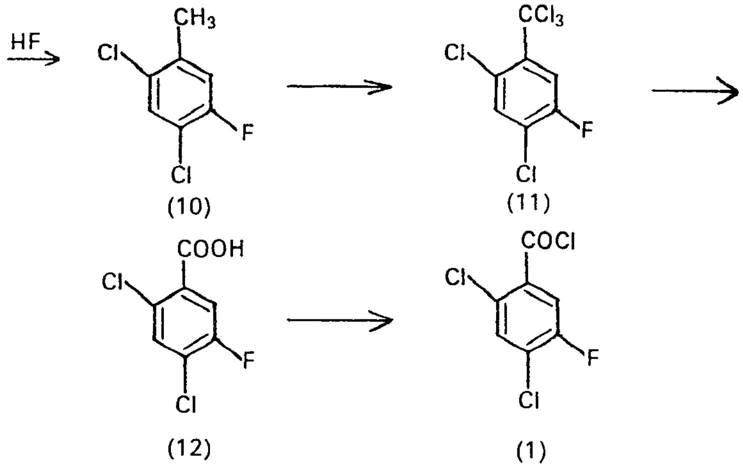


(10) (11)

(12) (1)

Danach wird 2,4-Dichlor-5-methyl-anilin (8) mit Hilfe von $NaNO_2$ diazotiert und das dabei entstehende Diazoniumsalz mit Dimethylamin in das Triazen (9) übergeführt.

Das Triazen (9) wird in überschüssiger wasserfreier HF gelöst. Dabei spaltet das Triazen in 2,4-Dichlor-5-methyl-diazoniumflorid und Dimethylamin. Ohne Zwischenisolierung wird diese Lösung thermisch bei 130–140° unter $N_2$-Abspaltung in 3-Fluor-4,6-dichlortoluol (10) gespalten. Ausbeute: 77% der Theorie.

Das 3-Fluor-4,6-dichlortoluol (10) wird in einem Temperaturbereich von 110 bis 160 °C unter UV-Bestrahlung zu 2,4-Dichlor-5-fluor-1-trichlormethylbenzol (11) chloriert.

Die Verseifung von (11) mit 95%iger Schwefelsäure führt zu 2,4-Dichlor-5-fluor-benzoesäure (12), die mit Thionylchlorid in das Carbonsäurechlorid (1) (Siedepunkt 121°/20 mbar; $n_D^{20}$ 1,5722) übergeht.

Auf analoge Weise werden die folgenden als Zwischenprodukte verwendeten Chinoloncarbonsäuren hergestellt:

7-Chlor-1-cyclopropyl-1,4-dihydro- 4-oxo-3-chinolincarbonsäure (VIb) (Schmelzpunkt 308 °C) aus 2,4-Dichlorbenzoylchlorid (J. Chem. Soc. 83, 1213 (1903) );

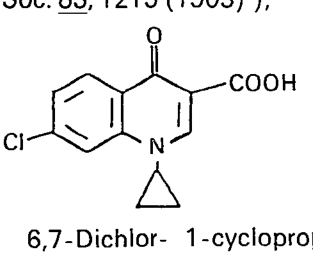

(VIb)

6,7-Dichlor- 1-cyclopropyl- 1,4-dihydro- 4-oxo-3-chinolincarbonsäure (VIc) (Schmelzpunkt 265 °C) aus 2,4,5-Trichlorbenzoylchlorid (Liebigs Ann. Chem. 152, 238 (1869) );

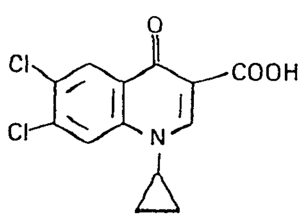

(VIc)

7-Chlor- 1-cyclopropyl-1,4- dihydro- 6-nitro-4- oxo-3-chinolincarbonsäure (VId) (Schmelzpunkt 265 bis 275 °C Zers.) aus 2,4-Dichlor- 5-nitro- benzoylchlorid (Liebigs Ann. Chem. 677, 8 (1964) ).

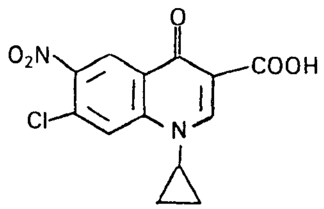

(VId)

Die erfindungsgemäss verwendbaren Verbindungen der Formel (III) sind bereits bekannt oder können nach bekannten Verfahren erhalten werden. Als Beispiele seien genannt:

Bromessigsäuremethylester, Bromessigsäureethylester, 2-Chlorpropionsäuremethylester, 3-Jodpropionsäurebenzylester, 4-Brombuttersäureethylester, 6-Jodhexansäurebenzylester, α-Bromphenylessigsäurebenzeylester, Bromessigsäure, Chloracetamid, N-Methyl-chloracetamid, N-Pentalchloracetamid, Chloracetonitril, α-Chloracetessigsäuremethylester, α-Bromacetessigsäureethylester, Brommalonsäurediethylester, Bromcyanessigsäureethylester, Brommalonsäurediamid, Brommalonsäuredinitril, Bromcyanessigsäureamid, 3-Brom-2-tertrhydrofuranon, Methoxymethylenmalonsäuredimethyleste, Ethoxymethylenmalonsäurediethylester, Methoxymethylenacetessigsäuremethylester, Ethoxymethylen malonsäuredinitril, Methoxymethylen-cyanessigsäuremethylester.

Die erfindungsgemäss verwendbaren Verbindungen der Formel (IV) sind bekannt. Als Beispiele seien genannt:

Acrylsäuremethylester, Acrylsäureethylester, Acrylsäurebutylester, Acrylsäurehexylester, Acrylsäurebenzylester, Methacrylsäuremethylester, Acrylnitril, 2-Chloracrylnitril, Methyl-vinyl-sulfon, Vinylphosphonsäurediethylester.

Die erfindungsgemäss verwendbaren Verbindungen der Formel (V) können nach den oben beschriebenen Methoden A und B erhalten werden.

Die Umsetzung von (II) mit (III) (Methode A) wird vorzugsweise in einem Verdünnungsmittel wie Dimethylsulfoxid, N,N-Dimethylformamid, Tetrahydrofuran, Sulfolan, Dioxan, Pyridin oder auch in Gemischen dieser Verdünnungsmittel bei Temperaturen von 0 °C bis 150 °C, vorzugsweise 30 °C bis 110 °C, vorgenommen.

Die Umsetzung kann bei Normaldruck, aber auch bei erhöhtem Druck, durchgeführt werden. Im allgemeinen arbeitet man bei Drucken zwischen etwa 1 und etwa 100 bar, vorzugsweise zwischen 1 und 10 bar.

Als Säurebinder können alle üblichen anorganischen und organischen Säurebindungsmittel verwendet werden. Hierzu gehören vorzugsweise die Alkalihydroxide, Alkalicarbonate, Pyridin und tert.-Amine wie Triethylamin, 1,4-Diazabicyclo-[2,2,2]octan. Die Umsetzung kann durch Zusatz von Kaliumiodid erleichtert werden.

Bei der Durchführung des erfindungsgemässen Verfahrens setzt man auf 1 Mol der Verbindung (II) 1 bis 4 Mol, vorzugsweise 1 bis 1,5 Mol der Verbindung (III) ein.

Die Umsetzung von (II) mit (IV) (Methode B) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Reaktionstemperaturen können in einem grösseren Bereich variiert werden. Im allgemeinen arbeitet man zwischen etwa 20 °C und etwa 150 °C, vorzugsweise zwischen 50 °C und 100 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemässen Verfahrens setzt man auf 1 Mol der Verbindung (II) 1–5 Mol, vorzugsweise 1–2 Mol, der Verbindung (IV) ein.

Die Umsetzung der Verbindung (V) zu den Dicarbonsäuren (Ib) (Methode C) erfolgt entweder in alkoholischer Natronlauge oder Kalilauge oder unter sauren Bedingungen in Mischungen von Schwefelsäure oder Chlorwasserstoff in Essigsäure und/oder Wasser. Die Hydrogenolyse von Benzylestern (V; R' = Benzyl) kann in Essigsäure in Gegenwart von Palladium-Katalysatoren erfolgen.

Man arbeitet im allgemeinen bei Temperaturen von 20 °C bis 160 °C, vorzugsweise bei 30 bis 140 °C.

Die Umsetzung kann bei Normaldruck, aber auch bei erhöhtem Druck, durchgeführt werden. Im allgemeinen arbeitet man bei Drucken zwischen etwa 1 und etwa 100 bar, vorzugsweise zwischen 1 und 10 bar.

Als neue antibakterielle Wirkstoffe seien im einzelnen genannt:

7-[4-(Methoxycarbonylmethyl)-1-piperazinyl]-1-cyclopropyl-6-fluor-1,4-dihydro-5-oxo-3-chinolincarbonsäure,

7-[4-(Ethoxycarbonylmethyl)-1-piperazinyl]-1-cyclopropyl-6-fluor-1,4-dihydro-4-oxo-3-chinolincarbonsäure,

7-[4-(2-Benzyloxycarbonylethyl)-1-piperazinyl]-1-cyclopropyl-6-fluor-1,4-dihydro-4-oxo-3-chinolincarbonsäure,

7-[4-(5-Benzyloxycarbonylpentyl)-1-piperazinyl]-1-cyclopropyl-6-fluor-1,4-dihydro-4-oxo-3-chinolincarbonsäure,

7-[4-(2-Methoxycarbonylethyl)-1-piperazinyl]-1-cyclopropyl-6-fluor-1,4-dihydro-4-oxo-3-chinolincarbonsäure,

7-[4-(2-Ethoxycarbonylethyl)-1-piperazinyl]-1-cyclopropyl-6-fluor-1,4-dihydro-4-oxo-3-chinolincarbonsäure,

7-[4-(2-Propyloxycarbonylethyl)-1-piperazinyl]-1-cyclopropyl-6-fluor-1,4-dihydro-4-oxo-3-chinolincarbonsäure,

7-[4-(2-n-Butoxycarbonylethyl)-1-piperazinyl]-1-cyclopropyl-6-fluor-1,4-dihydro-4-oxo-3-chinolincarbonsäure,

7-[4-(2-Cyanoethyl)-1-piperazinyl]-1-cyclopropyl-6-fluor-1,4-dihydro-4-oxo-3-chinolincarbonsäure,

7-[4-(3-Cyanopropyl)-1-piperazinyl]-1-cyclopropyl-6-fluor-1,4-dihydro-4-oxo-3-chinolincarbonsäure,

7-{4-[α-(Benzyloxycarbonyl)-benzyl]-1-piper-4-azinyl}-1-cyclopropyl-6-fluor-1,4-dihydro-4-oxo-3-chinolincarbonsäure,

7-[4-Carbamoylmethyl-1-piperazinyl]-1-cyclopropyl-6-fluor-1,4-dihydro-4-oxo-3-chinolincarbonsäure,

7-[4-Cyanomethyl-1-piperazinyl]-1-cyclopropyl-6-fluor-1,4-dihydro-4-oxo-3-chinolincarbonsäure,

7-[4-(N-Methylcarbamoylmethyl)-1-piperazinyl]-1-cyclopropyl-6-fluor-1,4-dihydro-4-oxo-3-chinolincarbonsäure,

7-[4-(N-Ethylcarbamoylmethyl)-1-piperazinyl]-1-cyclopropyl-6-fluor-1,4-dihydro-4-oxo-3-chinolincarbonsäure,

7-{4-[2-Oxo-1-(methoxycarbonyl)-1-propyl]-1-piperazinyl}-1-cyclopropyl-6-fluor-1,4-dihydro-4-oxo-3-chinolincarbonsäure,

7-[4-(2-Oxo-tetrahydrofuryl-3-)-1-piperazinyl]-1-cyclopropyl-6-fluor-1,4-dihydro-4-oxo-3-chinolincarbonsäure,

7-[4-(Carboxymethyl)-1-piperazinyl]-1-cyclopropyl-6-fluor-1,4-dihydro-4-oxo-3-chinolincarbonsäure,

7-[4-(2-Carboxyethyl)-1-piperazinyl]-1-cyclopropyl-6-fluor-1,4-dihydro-4-oxo-3-chinolincarbonsäure,

7-[4-(2-Carboxypropyl)-1-piperazinyl]-1-cyclopropyl-6-fluor-1,4-dihydro-4-oxo-3-chinolincarbonsäure,

7-[4-(3-Carboxypropyl)-1-piperazinyl]-1-cyclopropyl-6-fluor-1,4-dihydro-4-oxo-3-chinolincarbonsäure,

7-[4-(5-Carboxypentyl)-1-piperazinyl]-1-cyclopropyl-6-fluor-1,4-dihydro-4-oxo-3-chinolincarbonsäure,

7-[4-(α-Carboxybenzyl)-1-piperazinyl]-

1-cyclopropyl-6-fluor-1,4-dihydro-4-oxo-3-chinolincarbonsäure,
7-[4-(2-Chlor-2-cyanoethyl)-1-piperazinyl]-1-cyclopropyl-6-fluor-1,4-dihydro-4-oxo-3-chinolincarbonsäure,
7-[4-(2-Methylsulfonyl-ethyl)-1-piperazinyl]-1-cyclopropyl-6-fluor-1,4-dihydro-4-oxo-3-chinolincarbonsäure,
7-[4-(2-Diethoxyphosphonyl-ethyl)-1-piperazinyl]-1-cyclopropyl-6-fluor-1,4-dihydro-4-oxo-3-chinolincarbonsäure,
7-[4-(2-Methoxycarbonylethyl)-1-piperazinyl]-1-cyclopropyl-1,4-dihydro-6-nitro-4-oxo-3-chinolincarbonsäure,
7-[4-(2-Methoxycarbonylethyl)-1-piperazinyl]-6-chlor-1-cyclopropyl-1,4-dihydro-4-oxo-3-chinolincarbonsäure,
7-[4-(2-Methoxycarbonylethyl)-1-piperazinyl]-1-cyclopropyl-1,4-dihydro-4-oxo-3-chinolincarbonsäure,
7-[4-(2-Benzyloxycarbonylethyl)-3-methyl-1-piperazinyl]-1-cyclopropyl-6-fluor-1,4-dihydro-4-oxo-3-chinolincarbonsäure,
7-[4-(2-Methoxycarbonylethyl)-3,5-dimethyl-1-piperazinyl]-1-cyclopropyl-6-fluor-1,4-dihydro-4-oxo-3-chinolincarbonsäure.

Die erfindungsgemässen Verbindungen der Formel (I) können gegebenenfalls mit einer organischen oder anorganischen Säure in ein Salz übergeführt werden. Zur Salzbildung geeignete Säuren sind z.B. Halogenwasserstoffsäuren wie Salzsäure, Bromwasserstoffsäure, Jodwasserstoffsäure, Schwefelsäure, Essigsäure, Zitronensäure, Ascorbinsäure und Benzolsulfonsäure. Als Alkali- bzw. Erdalkalisalze sind vorzugsweise Natrium-, Kalium-, Calcium- und Magnesiumsalz geeignet.

Herstellungsbeispiele für die Ausgangsverbindungen:

Beispiel A

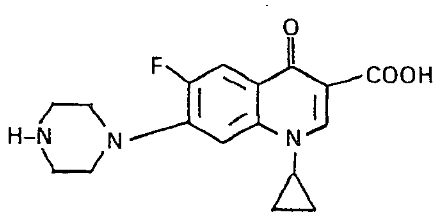

Ein Gemisch von 19,7 g 7-Chlor-1-cyclopropyl-6-fluor-1,4-dihydro-4-oxo-3-chinolincarbonsäure, 30,1 g wasserfreiem Piperazin und 100 ml Dimethylsulfoxid wird 2 Stunden auf 135 bis 140 °C erhitzt. Das Lösungsmittel wird im Feinvakuum abdestilliert, der Rückstand in $H_2O$ suspendiert, abgesaugt und mit Wasser gewaschen. Zur weiteren Reinigung wird das feuchte Rohprodukt mit 100 ml Wasser aufgekocht, bei Raumtemperatur abgesaugt, mit $H_2O$ gewaschen und im Vakuumtrockenschrank über $CaCl_2$ bei 100 °C bis zur Gewichtskonstanz getrocknet. Man erhält 19,6 g 1-Cyclopropyl-6-fluor-1,4-dihydro-4-oxo-7-(1-piperazinyl)-3-chinolincarbonsäure vom Zersetzungspunkt 255 bis 257 °C.

Die als Ausgangsmaterial verwendete 7-Chlor-1-cyclopropyl-6-fluor-1,4-dihydro-4-oxo-3-chinolincarbonsäure VIa wird wie folgt hergestellt:

24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester, 100 ml absolutem Ethanol und 400 ml wasserfreiem Ether zu, wobei ein heftiger Rückfluss zu beobachten ist. Nach dem Abklingen der Reaktion wird noch 2 Stunden zum Sieden erhitzt, mit Trockeneis/Aceton auf −5 °C bis −10 °C gekühlt und bei dieser Temperatur eine Lösung von 227,5 g 2,4-Dichlor-5-fluorbenzoylchlorid (1) in 100 ml absolutem Ether langsam zugetropft. Man rührt 1 Stunde bei 0 °C bis −5 °C, lässt über Nacht auf Raumtemperatur kommen und lässt unter Eiskühlung ein Gemisch von 400 ml Eiswasser und 25 ml konzentrierter Schwefelsäure zulaufen. Die Phasen werden getrennt und zweimal mit Ether nachextrahiert. Die vereinigten Etherlösungen werden mit gesättigter NaCl-Lösung gewaschen, mit $Na_2SO_4$ getrocknet und das Lösungsmittel im Vakuum abgezogen. Man erhält 349,5 g 2,4-Dichlor-5-fluor-benzoyl-malonsäure-diethylester (3) als Rohprodukt.

Eine Emulsion von 34,9 g rohem 2,4-Dichlor-5-fluor-benzoylmalonsäure-diethylester (3) in 50 ml Wasser wird mit 0,15 g p-Toluolsulfonsäure versetzt. Man erhitzt unter gutem Rühren 3 h zum Sieden, extrahiert die erkaltete Emulsion mehrmals mit Methylenchlorid, wäscht die vereinigten $CH_2Cl_2$-Lösungen einmal mit gesättigter NaCl-Lösung, trocknet mit $Na_2SO_4$ und destilliert das Lösungsmittel im Vakuum ab. Die Fraktionierung des Rückstands im Vakuum liefert 21,8 g 2,4-Dichlor-5-fluor-benzoyl-essigsäureethylester (4) vom Siedepunkt 127 bis 142 °C/0,09 mbar.

Ein Gemisch von 21,1 g 2,4-Dichlor-5-fluorbenzoyl-essigsäureethylester (4), 16,65 g o-Ameisensäureethylester und 18,55 g Acetanhydrid wird 2 Stunden auf 150 °C erhitzt. Dann werden im Wasserstrahlvakuum und zuletzt im Feinvakuum bei einer Badtemperatur von 120 °C die flüchtigen Bestandteile abdestilliert. Zurück bleiben 25,2 g roher 2-(2,4-Dichlor-5-benzoyl)-3-ethoxy-acrylsäureethylester (5). Er ist genügend rein für die weiteren Umsetzungen.

Eine Lösung von 24,9 g 2-(2,4-Dichlor-5-fluor-benzoyl)-3-ethoxy-acrylsäureethylester (5) in 80 ml Ethanol wird unter Eiskühlung und Rühren tropfenweise mit 4,3 g Cyclopropylamin versetzt. Wenn die exotherme Reaktion abgeklungen ist, wird noch 1 Stunde bei Raumtemperatur gerührt, das Lösungsmittel im Vakuum abgezogen und der Rückstand aus Cyclohexan/Petrolether umkristallisiert. Man erhält 22,9 g 2-(2,4-Dichlor-5-fluor-benzoyl)-3-cyclopropylamino-acrylsäureethylester (6) vom Schmelzpunkt 89 bis 90 °C.

Eine Lösung von 31,9 g 2-(2,4-Dichlor-5-fluor-benzoyl)-3-cyclopropylamino-acrylsäureethylester (6) in 100 ml wasserfreiem Dioxan wird unter Eiskühlung und Rühren portionsweise mit 3,44 g 80%igem Natriumhydrid versetzt. Dann wird 30 Minuten bei Raumtemperatur und 2 Stunden unter Rückfluss gerührt und das Dioxan im Vakuum abgezogen. Der Rückstand (40,3 g) wird in 150 ml Wasser suspendiert, mit 6,65 g Ätzkali versetzt und 1,5 h refluxiert. Man filtriert die warme Lösung und wäscht mit $H_2O$ nach. Dann wird mit halbkonzentrierter Salzsäure unter Eiskühlung auf pH=1–2 angesäuert, der Niederschlag abgesaugt, mit Wasser gewaschen und im Vakuum bei 100 °C getrocknet. Man erhält auf diese Weise 27,7 g 7-Chlor-1-cyclopropyl-6-fluor-1,4-dihydro-4-oxo- 3-chinolincarbonsäure VIa vom Schmelzpunkt 234 bis 237 °C.

Beispiel B

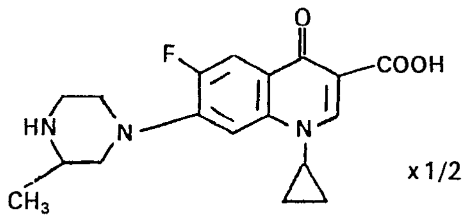

x 1/2 $H_2O$

Ein Gemisch aus 2,8 g (0,01 Mol) 7-Chlor-1-cyclopropyl-6-fluor -1,4-dihydro-4-oxo- 3-chinolincarbonsäure und 5,1 g (0,051 Mol) 2-Methylpiperazin in 6 ml Dimethylsulfoxid wird 2 Stunden auf 140 °C erhitzt. Anschliessend wird das Lösungsmittel im Hochvakuum abdestilliert, der Rückstand mit 6 ml heissem Wasser versetzt und 1 Stunde auf 95 °C gehalten. Man kühlt mit Eis, saugt den ausgefallenen Niederschlag ab, wäscht mit etwas Wasser und löst ihn in einer Mischung von 0,8 ml Essigsäure und 10 ml Wasser bei 90 bis 100 °C auf. Das Filtrat wird mit Kalilauge (0,75 g KOH in 0,7 ml Wasser) auf pH 8 gebracht und der ausgefallene Niederschlag aus Methanol umkristallisiert. Man erhält 1,8 g (52% der Theorie) 1-Cyclopropyl-6-fluor- 1,4-dihydro-4-oxo-7- (3-methyl-1-piperazinyl) -3-chinolincarbonsäure- semihydrat mit einem Zersetzungspunkt von 230 bis 232 °C.

Beispiel C

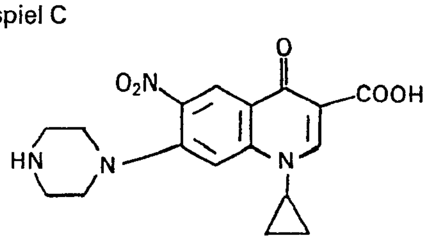

Eine Mischung von 9,3 g (0,03 Mol) 7-Chlor-1-cyclopropyl- 1,4-dihydro- 6-nitro- 4-oxo- 3-chinolincarbonsäure und 12,9 g (0,15 mol) Piperazin wird in 60 ml Dimethyulsulfoxid 15 Minuten auf 120 °C erwärmt. Aus der heissen Lösung fällt nach kurzer Zeit ein Niederschlag aus. Man engt im Hochvakuum ein, verrührt mit 30 ml Wasser und erhitzt noch 30 Minuten auf 95 °C. Mit 2n-Salzsäure wird die Mischung auf pH 8 eingestellt, der Niederschlag abgesaugt und mit Wasser und Methanol gewaschen. Man isoliert 5,8 g (54% der Theorie) 1-Cyclopropyl-1,4-dihydro-6- nitro-4-oxo-7- (1-piperazinyl) -3-chinolincarbonsäure mit einem Zersetzungspunkt von 296 bis 298 °C.

Beispiel D

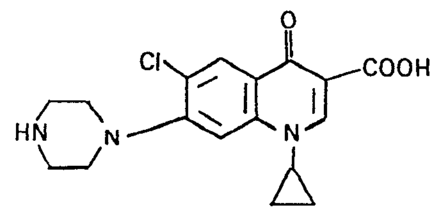

Man setzt analog Beispiel C 6,7-Dichlor-1-cyclopropyl-1,4- dihydro-4-oxo-3- chinoloncarbonsäure zu 1-Cyclopropyl-6-chlor-1,4- dihydro-4-oxo-7- (1-piperazinyl) -3-chinolincarbonsäure mit einem Zersetzungspunkt von 295 bis 298 °C um.

Beispiel E

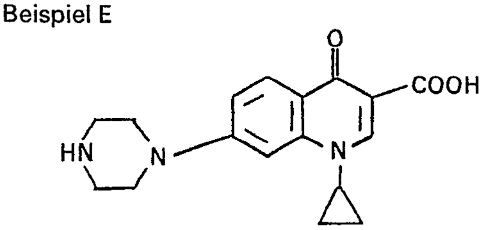

Man setzt analog Beispiel C 7-Chlor-1-cyclopropyl-1,4-dihydro-4-oxo-3- chinolincarbonsäure mit Piperazin zu 1-Cyclopropyl-1,4- dihydro-4- oxo- 7- (1-piperazinyl) -3-chinolincarbonsäure mit einem Zersetzungspunkt von 298 bis 300 °C um.

Herstellungsbeispiele für die erfindungsgemässen Endprodukte:

Beispiel 1

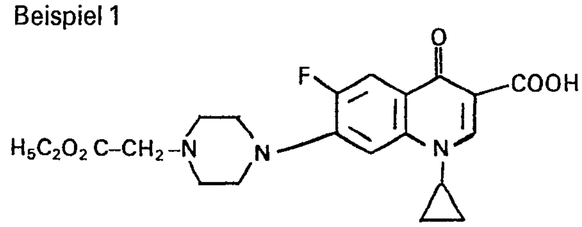

3,3 g (0,01 Mol) 1-Cyclopropyl-6-fluor -1,4-dihydro-4-oxo-7- (1-piperazinyl) -3-chinolincarbonsäure werden in 50 ml Dimethylformamid mit 2,5 g (0,015 Mol) Bromessigsäureethylester, 2,1 g (0,02 Mol) Triethylamin und 2,5 g Kaliumjodid 5 Stunden auf 90 °C erhitzt. Die Reaktionsmischung wird in 30 ml Wasser eingegossen, der Niederschlag abgesaugt, mit Wasser gewaschen und aus Methanol umkristallisiert. Man erhält 2,5 g 1-Cyclopropyl-6-fluor-7- [4-(ethoxycarbonylmethyl) -1-piperazinyl] -1,4-dihydro-4-oxo- 3-chinolincarbonsäure mit einem Schmelzpunkt von 192 bis 194 °C.

Analog Beispiel 1 werden folgende Verbindungen erhalten:

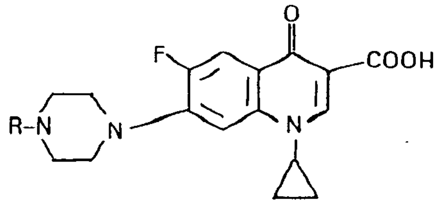

| Beispiel | R | Schmelzpunkt (°C) |
|---|---|---|
| 2 | $C_6H_5CH_2O-CO-CH-$<br>$\vert$<br>$C_6H_5$ | 170 (Zers.) |
| 3 | $H_2N-CO-CH_2-$ | 254 (Zers.) |
| 4 | $NC-CH_2-$ | 166 (Zers.) |

Beispiel 5

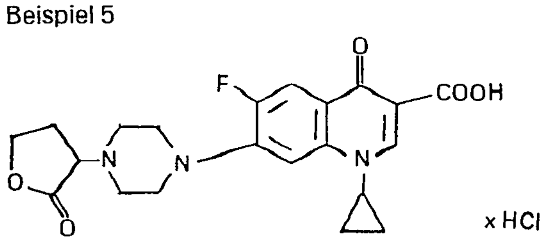

x HCl

Man arbeitet analog Beispiel 1 mit α-Brombutyrolacton als Alkylierungsmittel, behandelt das Reaktionsprodukt mit verdünnter Salzsäure und erhält 1-Cyclopropyl-6-fluor -1,4-dihydro-4-oxo-7- [4-(2-oxo-tetrahydrofuryl-3-) -1-piperazinyl] -3-chinolincarbonsäure- hydrochlorid mit einem Zersetzungspunkt von 270 °C.

Massenspektrum: m/e 415 ($M^+$), 371, 342, 331, 301, 298, 289, 287, 275, 257, 245, 229, 36 (100% HCl).

Beispiel 6

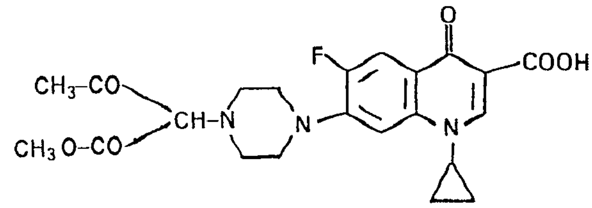

6,6 g (0,02 Mol) 1-Cyclopropyl-6-fluor -1,4-dihydro-4-oxo-7- (1-piperazinyl) -3-chinolincarbonsäure werden mit 4,5 g 2-Chloracetessigsäuremethylester und 4,2 g Triethylamin in 100 ml Dimethylformamid 3 Stunden auf 80 °C erhitzt. Anschliessend wird die Lösung im Vakuum eingeengt, mit 50 ml Wasser verrührt, das erhaltene Festprodukt mit Wasser und Methanol gewaschen und aus Glykolmonomethylether umkristallisiert. Man isoliert 3,9 g (44% der Theorie) 1-Cyclopropyl- 6-fluor -1,4-dihydro- 4-oxo-7 {4- [2- oxo -1- (methoxycarbonyl) -1- propyl]-1-piperazinyl} -3- chinolincarbonsäure mit einem Zersetzungspunkt von 224 bis 228 °C.

Beispiel 7

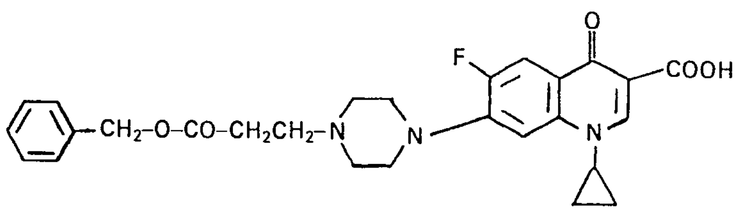

XHJ

3,3 g (0,01 Mol) 1-Cyclopropyl-6-fluor -1,4-dihydro-4-oxo-7- (1-piperazinyl) -3-chinolincarbonsäure werden in 50 ml Dimethylformamid mit 5,8 g 3-Jodpropionsäurebenzylester und 2,1 g Triethylamin 2 1/2 Stunden unter Rühren auf 70 bis 80 °C erhitzt. Die Lösung wird im Vakuum eingeengt, mit 30 ml Wasser versetzt und auf pH 5 eingestellt. Der Niederschlag wird abgesaugt und mit Methanol ausgekocht, wobei 2,8 g 7-[4-(2-Benzyloxycarbonylethyl) -1-piperazinyl] -1-cyclopropyl-6-fluor- 1,4-dihydro-4-oxo-3- chinolincarbonsäure-hydrojodid mit einem Zersetzungspunkt von 206 bis 210 °C erhalten werden.

Der als Ausgangsstoff verwendete 3-Jodpropionsäurebenzylester wird auf folgendem Weg erhalten:

99 g 3-Chlorpropionsäurebenzylester werden mit 90 g Natriumiodid in 460 ml Aceton 1 Tag zum Rückfluss erhitzt. Das Reaktionsgemisch wird eingeengt, mit 200 ml Methylenchlorid versetzt und mit 3×100 ml Wasser gewaschen. Nach dem Trocknen mit Natriumsulfat wird eingeengt und der Rückstand im Hochvakuum destilliert; Ausbeute: 91 g 3-Jodpropionsäurebenzylester mit einem Siedepunkt von 105 bis 108 °C/0,1 mmHg.

Beispiel 8

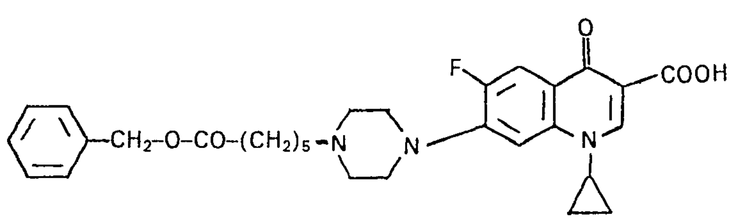

Man arbeitet analog Beispiel 7 mit 6-Jodhexansäurebenzylester und erhält 7-[4-(5-Benzyloxycarbonylpentyl) -1-piperazinyl] -1-cyclopropyl-6- fluor -1,4-dihydro- 4-oxo-3- chinolincarbonsäure mit einem Schmelzpunkt von 176 bis 178 °C.

Der als Ausgangsprodukt verwendete 6-Jodhexansäurebenzylester wird auf folgendem Weg erhalten:

46,5 g (0,3 Mol) 6-Chlorhexansäure und 35,6 g Benzylalkohol werden in 500 ml Toluol in Gegenwart von 1 g p-Toluolsulfonsäure am Wasserabscheider erhitzt. Nach beendeter Reaktion wäscht man mit 5% Natriumbicarbonatlösung und Wasser, trocknet mit Natriumsulfat, engt ein und destilliert den Rückstand, wobei 61,5 g (85% der Theorie) 6-Chlorhexansäurebenzylester, Siedepunkt 163 bis 165 °C/4 mmHg.

60 g (0,25 Mol) 6-Chlorhexansäurebenzylester werden mit 45 g Natriumjodid in 230 ml Aceton 5 Stunden unter Rückfluss erhitzt. Die Suspension wird eingeengt, mit 300 ml Methylenchlorid versetzt und mit 2×200 ml Wasser gewaschen. Die organische Phase trocknet man mit Natriumsulfat, engt ein und destilliert den Rückstand in einer Kugelrohrdestillationsapparatur. Bei 220 bis 230 °C (Ofentemperatur)/ 0,4 mmHg gehen 63,8 g (77% der Theorie) 6-Jodhexansäurebenzylester über.

Beispiel 9

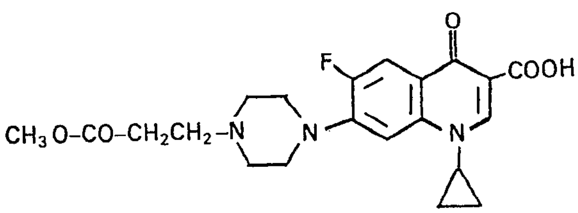

Eine Mischung von 3,31 g (0,01 Mol) 1-Cyclopropyl- 6-fluor- 1,4-dihydro- 4-oxo- 7-(1-piperazinyl) -3-chinolincarbonsäure und 5 g (0,058 Mol) Acrylsäuremethylester in 50 ml Ethanol wird 2 Stunden unter Rückfluss erhitzt. Die Lösung wird in 10 ml Wasser eingegossen, der Niederschlag abgesaugt, mit Methanol gewaschen und aus Glykolmonomethylether umkristallisiert. Man erhält 2,9 g (70% der Theorie) 1-Cyclopropyl- 6-fluor -1,4-dihydro- 4-oxo- 7-[4-(2-methoxycarbonylethyl) -1-piperazinyl] -3-chinolincarbonsäure mit einem Zersetzungspunkt von 192 bis 194 °C.

Analog Beispiel 9 werden folgende Verbindungen erhalten:

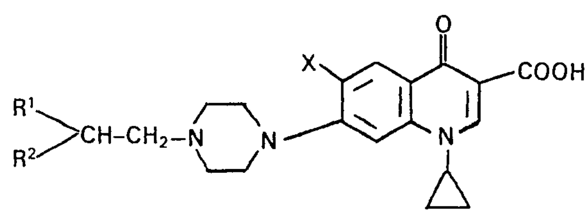

| Beispiel | $R^1$ | $R^2$ | X | Schmelzpunkt (°C) |
|---|---|---|---|---|
| 10 | $C_2H_5O-CO$ | H | F | 142 (Zers.) |
| 11 | $C_4H_9O-CO$ | H | F | 141 (Zers.) |
| 12 | $C_6H_5CH_2O-CO$ | H | F | 140 |
| 13 | $CH_3O-CO$ | H | Cl | 183 |
| 14 | CN | H | F | 255 (Zers.)$^{+)}$ |
| 15 | CN | Cl | F | 202 (Zers.)$^{++)}$ |
| 16 | $CH_3-SO_2$ | H | F | 258 (Zers.) |

$^{+)}$ Die 7-[4-(2-Cyanoethyl) -1-piperazinyl)]-1-cyclopropyl- 6-fluor- 1,4- dihydro- 4-oxo-3-chinolincarbonsäure liegt nach dem $^1$H-Kernresonanzspektrum im Gemisch mit ~15% 7-[4-(1-Cyanoethyl) -1-piperazinyl] -1-cyclopropyl-6-fluor -1,4-dihydro-4-oxo-3- chinolincarbonsäure vor.

$^{++)}$ Massenspektrum: m/e 382 ($M^+$–HCl), 338 (382–$CO_2$), 331, 289, 287, 245, 218, 154, 152, 44 ($CO_2$), 36 (100%, HCl)

Beispiel 17

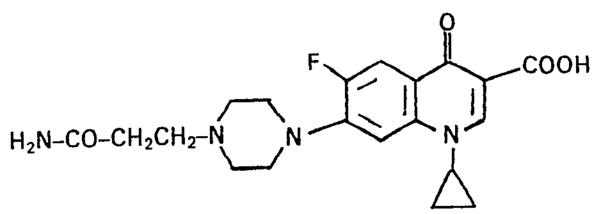

Eine Mischung von 3,31 g (0,01 Mol) 1-Cyclopropyl-6-fluor -1,4-dihydro-4-oxo-7- (1-piperazinyl) -3-chinolincarbonsäure und 4,2 g (0,058 Mol) Acrylamid in 50 ml Dimethylformamid wird 6 Stunden auf 140 °C erhitzt. Die Suspension wird im Hochvakuum eingeengt, der Rückstand mit Wasser verrührt und aus Glykolmonomethylether umkristallisiert. Man erhält 2 g (50% der Theorie) 7-[4-(2-Carbamoylethyl) -1-piperazinyl] -1-cyclopropyl- 6-fluor- 1,4-dihydro-4-oxo-3- chinolincarbonsäure vom Zersetzungspunkt 283 bis 286 °C.

Beispiel 18

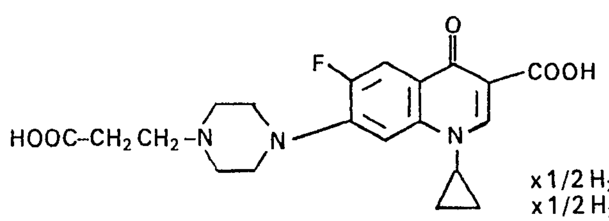

x 1/2 $H_2SO_4$
x 1/2 $H_2O$

2,9 g der Verbindung des Beispiel 9 werden in einer Mischung von 14 ml Essigsäure und 9,5 ml Wasser gelöst und mit 1,4 ml konzentrierter Schwefelsäure versetzt. Man erhitzt 1,5 Stunden auf 150 bis 160 °C und giesst die Mischung in 90 ml Wasser ein. Der Niederschlag wird abgesaugt, mit Wasser und Methanol gewaschen und getrocknet. Man isoliert 2,3 g (72% der Theorie) 7-[4-(2-Carboxyethyl) -1-piperazinyl] -1-cyclopropyl-6-fluor -1,4-dihydro-4-oxo-3- chinolincarbonsäure-semisulfat-semihydrat vom Zersetzungspunkt 258 bis 261 °C.

$C_{20}H_{22}FN_3O_5 \cdot 1/2\ H_2SO_4 \cdot 1/2\ H_2O$ (461,4)

| | | | | |
|---|---|---|---|---|
| berechnet: | C 52,06 | H 5,24 | N 9,11 | S 3,47 |
| gefunden: | C 51,7 | H 5,3 | N 9,1 | S 3,9 |

Analog Beispiel 12 werden folgende Verbindungen erhalten:

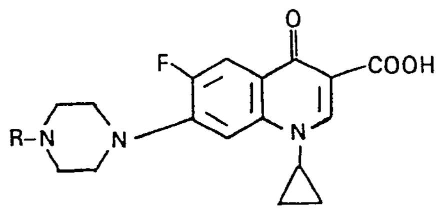

| Beispiel | R | Schmelzpunkt ( °C) |
|---|---|---|
| 19 | $HOOC-CH_2- \times 2^1/_2\ H_2O$ | 276 (Zers.)[1] |
| 20 | $HOOC-(CH_2)_5- \times 1/2\ H_2SO_4 \times 1/2\ H_2O$ | 254 (Zers.) |
| 21 | $HOOC-\underset{C_6H_5}{CH}- \times H_2O$ | 214 (Zers.)[2] |

[1] Das Reaktionsprodukt (als Sulfat) wurde in verdünnter Natronlauge gelöst und mit verdünnter Salzsäure bei pH 5 als Betain ausgefällt.

[2] Das auf Wasser gegebene Reaktionsgemisch wird mit verdünnter Natronlauge auf pH 4 eingestellt und das Betain isoliert.

Beispiel 22

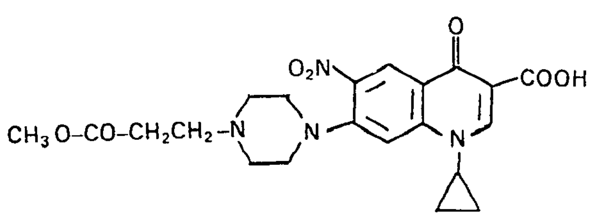

537 g (0,0015) 1-Cyclopropyl-1,4-dihydro-6-nitro-4-oxo-7- (1-piperazinyl) -3-chinolincarbonsäure werden in einer Mischung aus 7,5 ml Glykolmonomethylether und 3 ml Dimethylsulfoxid mit 2 g Acrylsäuremethylester 8 Stunden unter Rückfluss erhitzt. Die Lösung wird mit 10 ml Wasser versetzt, der Niederschlag abgesaugt, mit Methanol gewaschen und getrocknet. Man erhält 0,5 g 1-Cyclopropyl-1,4-dihydro-7- [4-(2-ethoxycarbonylethyl) -1-piperazinyl] -6-nitro-4-oxo-3- chinolincarbonsäure mit einem Zersetzungspunkt von 208 bis 211 °C.

Beispiel 23

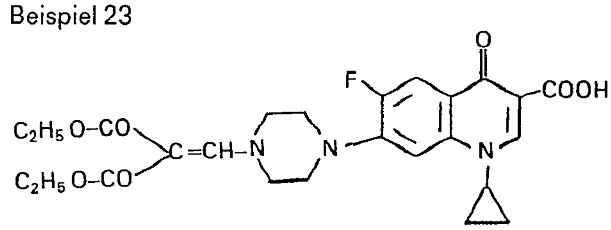

In einer Mischung aus 0,4 g Natriumhydroxid in 5 ml Wasser und 25 ml Dioxan werden 3,3 g 1-Cyclopropyl-6- fluor-1,4-dihydro- 4-oxo-7-(1-piperazinyl) -3-chinolincarbonsäure mit 2,8 g Ethoxymethylenmalonsäurediethylester 5 Stunden bei Raumtemperatur gerührt. Man lässt über Nacht stehen, filtriert von wenig Ungelöstem ab und engt das Filtrat ein. Der Rückstand wird in ca. 30 ml Wasser aufgenommen, mit verdünnter Salzsäure auf pH 4 eingestellt und der ausgefallene Niederschlag sofort abgesaugt und mit Wasser gewaschen. Man erhält ein schmieriges Produkt, das sich durch Verrühren mit Isopropanol verfestigt.

Ausbeute: 2,4 g (48% der Theorie) 1-Cyclopropyl-7- [4-(2,2-Diethoxycarbonyl- vinylen)- 1-piperazinyl] -6-fluor- 1,4-dihydro-4-oxo-3- chinolincarbonsäure mit einem Zersetzungspunkt von 184 bis 188 °C.

Beispiel 24

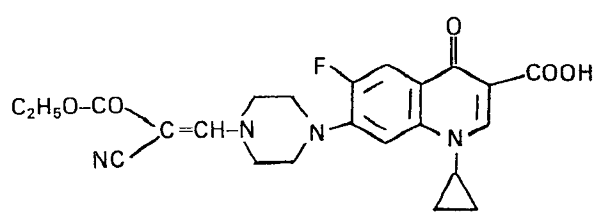

Man arbeitet analog Beispiel 23, jedoch mit 2,2 g Ethoxymethylencyanessigsäureethylester und erhält 2,35 g 1-Cyclopropyl-7- [3-(2-cyano-2- ethoxycarbonylvinylen) -1-piperazinyl]-6-fluor-1,4-dihydro-4- oxo-3-chinolincarbonsäure mit einem Zersetzungspunkt von 245 bis 255 °C.

Beispiel 25

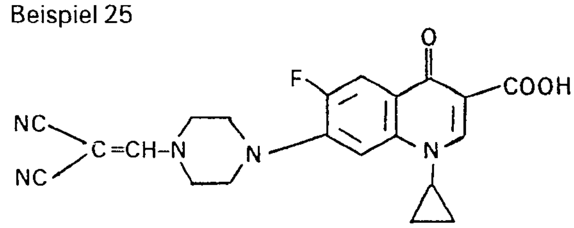

Man arbeitet analog Beispiel 23, jedoch mit 1,6 g Ethoxymethylenmalonsäuredinitril und erhält 4 g 1-Cyclopropyl-7-[4-(2,2-dicyano-viny-

len) -1- piperazinyl] -6- fluor- 1,4-dihydro-4-oxo-3- chinolincarbonsäure als schwerlösliches Produkt, das mit Ethanol gewaschen wurde. Zersetzungspunkt 275 bis 283 °C.

Massenspektrum: m/e = 363 ($M^+$ $-CO_2$), 362 ($M^+$ $-COOH$), 315, 287, 245, 44 (100%, $CO_2$).

Beispiel 26

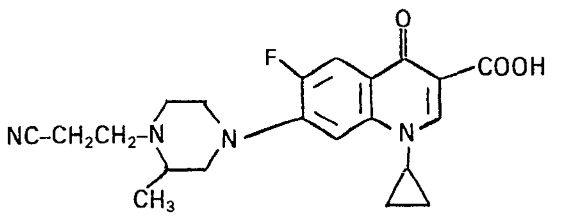

Man setzt analog Beispiel 9 3,45 g 1-Cyclopropyl-6-fluor-1,4- dihydro-4-oxo-7- (3-methyl-1-piperazinyl) -3-chinolincarbonsäure (Beispiel B) mit 4,5 g Acrylnitril um und erhält 3 g 7-[4-(2-Cyanoethyl) -3-methyl-1-piperazinyl] -1-cyclopropyl- 6-fluor- 1,4-dihydro- 4-oxo-3- chinolincarbonsäure mit einem Schmelzpunkt von 203–206 °C.

$C_{21}H_{23}FN_4O_3$ (398,4)

| | | | |
|---|---|---|---|
| berechnet: | C 63,3 | H 5,8 | N 14,1 |
| gefunden: | C 63,0 | H 5,9 | N 13,8 |

Die erfindungsgemässen Verbindungen haben gute Wirkungen gegen grampositive und gramnegative Bakterien. In der nachstehenden Tabelle sind die minimalen Hemmkonzentrationen für erfindungsgemässe Verbindungen bei einigen Bakterien angegeben. Sie wurden erhalten im Agarverdünnungstest mit Hilfe von Multipoint-Inokulator (Denley) auf Isosensitest-Agar.

| Stamm | Beispiel 6 | Beispiel 15 |
|---|---|---|
| E. coli Neumann | ≤0,015 | ≤0,015 |
| Klebsiella 8085 | ≤0,015 | ≤0,015 |
| Proteus 1017 | ≤0,015 | ≤0,015 |
| Staph. 133 | 1 | 0,5 |
| Pseudom. Walther | 0,5 | 0,5 |

**Patentansprüche**

1. Chinoloncarbonsäuren der Formel (I)

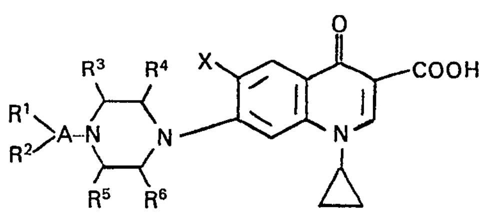 (I)

in der

A für geradkettiges oder verzweigtes Alkylen mit 1 bis 6 Kohlenstoffatomen oder einen Rest >C=CH–,

$R^1$ für Alkoxycarbonyl mit 1 bis 6 Kohlenstoffatomen im Alkylteil, Benzyloxycarbonyl, Carboxy, gegebenenfalls durch 1 oder 2 Methyl- oder Ethylreste substituiertes Carbamoyl, Cyano, Dialkoxyphosphonyl oder Alkylsulfonyl mit 1 bis 4 Kohlenstoffatomen im Alkylteil,

$R^2$ für Wasserstoff, Alkoxycarbonyl mit 1 bis 6 Kohlenstoffatomen im Alkylteil, Benzyloxycarbonyl, Carbamoyl, Cyano, Chlor, Acetyl, Alkyl mit 1 bis 3 Kohlenstoffatomen oder Phenyl steht, wobei $R^1$ und $R^2$ auch gemeinsam mit dem Kohlenstoffatom, das sie substituieren, einen 2-Oxotetrahydrofurylring bilden können und

$R^3$, $R^4$, $R^5$ und $R^6$ gleich oder verschieden sein können und für Wasserstoff, Methyl, Ethyl, n- oder i-Propyl stehen, und

X für Wasserstoff, Halogen, vorzugsweise Fluor oder Chlor, oder Nitro steht, und deren pharmazeutisch verwendbare Säureadditions-, Alkali- und Erdalkalisalze und Hydrate.

2. Chinoloncarbonsäuren der Formel (I) in Anspruch 1, in der

A geradkettiges oder verzweigtes Alkylen mit 1 bis 5 C-Atomen oder >C=CH–,

$R^1$ Alkoxycarbonyl mit 1 bis 5 C-Atomen im Alkylteil, Benzyloxycarbonyl, Carboxy, gegebenenfalls durch 1 oder 2 Methyl- oder Ethylreste substituiertes Carbamoyl, Cyano, Methyl- oder Ethylsulfonyl,

$R^2$ Wasserstoff, Alkoxycarbonyl mit 1 bis 5 C-Atomen im Alkylteil, Benzyloxycarbonyl, Carbamoyl, Cyano, Chlor, Acetyl, Alkyl mit 1 bis 2 Kohlenstoffatomen, Phenyl, wobei $R^1$ und $R^2$ auch gemeinsam mit dem C-Atom, das sie substituieren, einen 2-Oxo-tetrahydrofurylring bilden können,

$R^3$, $R^4$, $R^5$ und $R^6$ Wasserstoff, Methyl oder Ethyl und

X Wasserstoff, Fluor oder Nitro bedeuten.

3. Chinoloncarbonsäuren der Formel (I) in Anspruch 1, in der

A geradkettiges Alkylen mit 1 bis 5 C-Atomen oder >C=CH–,

$R^1$ Alkoxycarbonyl mit 1 bis 4 C-Atomen im Alkylteil, Benzyloxycarbonyl, Carboxy, Carbamoyl, Cyano, Methylsulfonyl,

$R^2$ Wasserstoff, Alkoxycarbonyl mit 1 bis 3 C-Atomen im Alkylteil, Cyano, Chlor, Acetyl, Phenyl, wobei $R^1$ und $R^2$ auch gemeinsam mit dem C-Atom, das sie substituieren, einen 2-Oxotetrahydro-3-furylring bilden können,

$R^3$ Wasserstoff, Methyl, Ethyl,

$R^4$ Wasserstoff,

$R^5$ Wasserstoff, Methyl,

$R^6$ Wasserstoff, und

X Wasserstoff, Fluor, Chlor, Nitro bedeuten.

4. 1-Cyclopropyl-6- fluor-1,4- dihydro-4-oxo-7- {4- [2- oxo-1- (methoxycarbonyl)-1-propyl]-1-piperazinyl} 3-chinolincarbonsäure.

5. 7- [4- (2-Chlor- 2-cyano-ethyl) -1-piperazinyl] -1-cyclopropyl- 6-fluor- 1,4-dihydro-4-oxo-3- chinolincarbonsäure.

6. Verwendung von Chinoloncarbonsäuren der Formel (I)

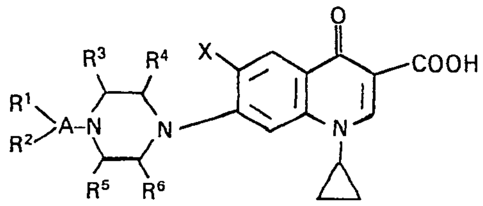

(I)

in der

A für geradkettiges oder verzweigtes Alkylen mit 1 bis 6 Kohlenstoffatomen oder einen Rest $>C=CH-$,

$R^1$ für Alkoxycarbonyl mit 1 bis 6 Kohlenstoffatomen im Alkylteil, Benzyloxycarbonyl, Carboxy, gegebenenfalls durch 1 oder 2 Methyl- oder Ethylreste substituiertes Carbamoyl, Cyano, Dialkoxyphosphonyl oder Alkylsulfonyl mit 1 bis 4 Kohlenstoffatomen im Alkylteil,

$R^2$ für Wasserstoff, Alkoxycarbonyl mit 1 bis 6 Kohlenstoffatomen im Alkylteil, Benzyloxycarbonyl, Carbamoyl, Cyano, Chlor, Acetyl, Alkyl mit 1 bis 3 Kohlenstoffatomen oder Phenyl steht, wobei $R^1$ und $R^2$ auch gemeinsam mit dem Kohlenstoffatom, das sie substituieren, einen 2-Oxotetrahydrofurylring bilden können und

$R^3$, $R^4$, $R^5$ und $R^6$ gleich oder verschieden sein können und für Wasserstoff, Methyl, Ethyl, n- oder i-Propyl stehen, und

X für Wasserstoff, Halogen, vorzugsweise Fluor oder Chlor, oder Nitro steht, und deren pharmazeutisch verwendbaren Säureadditions-, Alkali- und Erdalkalisalzen und Hydraten zur Herstellung von Arzneimitteln.

7. Verfahren zur Herstellung von Chinoloncarbonsäuren der Formel (I)

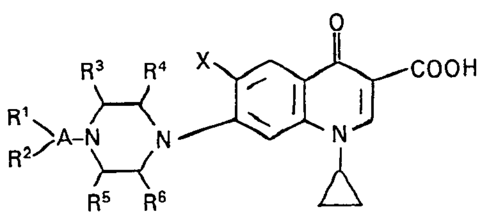

(I)

in der

A für geradkettiges oder verzweigtes Alkylen mit 1 bis 6 Kohlenstoffatomen oder einen Rest $>C=CH-$,

$R^1$ für Alkoxycarbonyl mit 1 bis 6 Kohlenstoffatomen im Alkylteil, Benzyloxycarbonyl, Carboxy, gegebenenfalls durch 1 oder 2 Methyl- oder Ethylreste substituiertes Carbamoyl, Cyano, Dialkoxyphosphonyl oder Alkylsulfonyl mit 1 bis 4 Kohlenstoffatomen im Alkylteil,

$R^2$ für Wasserstoff, Alkoxycarbonyl mit 1 bis 6 Kohlenstoffatomen im Alkylteil, Benzyloxycarbonyl, Carbamoyl, Cyano, Chlor, Acetyl, Alkyl mit 1 bis 3 Kohlenstoffatomen oder Phenyl steht, wobei $R^1$ und $R^2$ auch gemeinsam mit dem Kohlenstoffatom, das sie substituieren, einen 2-Oxotetrahydrofurylring bilden können und

$R^3$, $R^4$, $R^5$, und $R^6$ gleich oder verschieden sein können und für Wasserstoff, Methyl, Ethyl, n- oder i-Propyl stehen, und

X für Wasserstoff, Halogen, vorzugsweise Fluor oder Chlor, oder Nitro steht, und deren pharmazeutisch verwendbaren Säureadditions-, Alkali- und Erdalkalisalzen und Hydraten, dadurch gekennzeichnet, dass man entweder

a) Verbindungen der Formel (II)

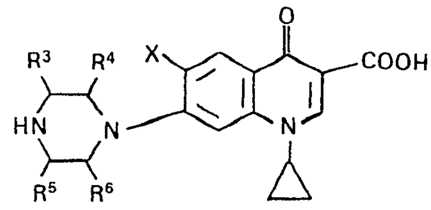

(II)

in welcher

X, $R^3$, $R^4$, $R^5$ und $R^6$ die oben angegebene Bedeutung haben, mit Verbindungen der Formel (III)

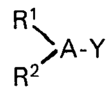

(III)

in welcher

$R^1$, $R^2$ und A die oben angegebene Bedeutung haben und

Y für Halogen, vorzugsweise Chlor, Brom oder Iod, $CH_3O-SO_2-O$, $C_2H_5O-SO_2-O$, Methoxy oder Ethoxy steht, umsetzt, oder dass man

b) Verbindungen der Formel (II) mit Verbindungen der Formel (IV)

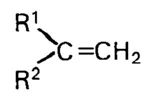

(IV)

in welcher

$R^1$ und $R^2$ die oben angegebene Bedeutung haben, umsetzt, oder dass man

c) Verbindungen der Formel (V)

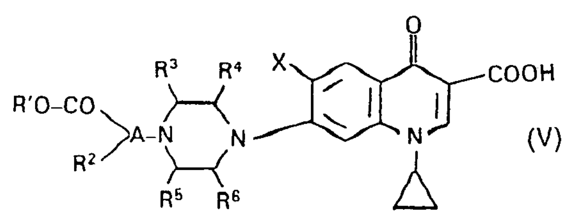

(V)

in welcher

$R^2$, $R^3$, $R^4$, $R^5$, $R^6$ und X die oben angegebene Bedeutung haben und

R' für Alkyl mit 1 bis 6 Kohlenstoffatomen oder Benzyl steht, unter alkalischen oder sauren Bedingungen oder im Falle von R'=Benzyl auch unter hydrogenolytischen Bedingungen umsetzt.

8. Arzneimittel, gekennzeichnet durch einen Gehalt an mindestens einer Chinoloncarbonsäure der Formel (I) in Anspruch 1.

9. Chinoloncarbonsäuren der Formel (I)

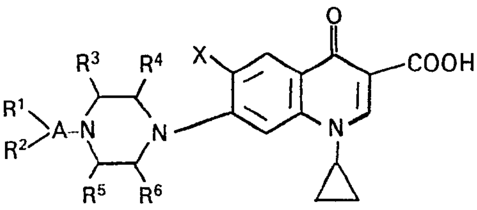

(I)

in der

A für geradkettiges oder verzweigtes Alkylen mit 1 bis 6 Kohlenstoffatomen oder einen Rest >C=CH–,

$R^1$ für Alkoxycarbonyl mit 1 bis 6 Kohlenstoffatomen im Alkylteil, Benzyloxycarbonyl, Carboxy, gegebenenfalls durch 1 oder 2 Methyl- oder Ethylreste substituiertes Carbamoyl, Cyano, Dialkoxyphosphonyl oder Alkylsulfonyl mit 1 bis 4 Kohlenstoffatomen im Alkylteil,

$R^2$ für Wasserstoff, Alkoxycarbonyl mit 1 bis 6 Kohlenstoffatomen im Alkylteil, Benzyloxycarbonyl, Carbamoyl, Cyano, Chlor, Acetyl, Alkyl mit 1 bis 3 Kohlenstoffatomen oder Phenyl steht, wobei $R^1$ und $R^2$ auch gemeinsam mit dem Kohlenstoffatom, das sie substituieren, einen 2-Oxotetrahydrofurylring bilden können und

$R^3$, $R^4$, $R^5$ und $R^6$ gleich oder verschieden sein können und für Wasserstoff, Methyl, Ethyl, n- oder i-Propyl stehen, und

X für Wasserstoff, Halogen, vorzugsweise Fluor oder Chlor, oder Nitro steht, und deren pharmazeutisch verwendbare Säureadditions-, Alkali- und Erdalkalisalze und Hydrate, zur Anwendung in einem Verfahren zur therapeutischen Behandlung des menschlichen oder tierischen Körpers.

**Revendications**

1. Acides quinolone carboxyliques de formule (I)

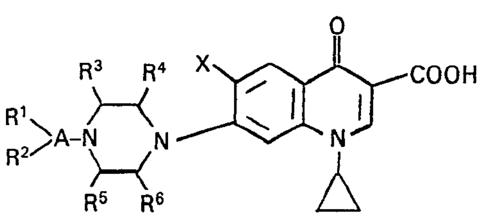

(I)

dans laquelle

A représente un alkylène à chaîne droite ou ramifiée avec 1 à 6 atomes de carbone ou un reste >C=CH–,

$R^1$ représente un alcoxycarbonyle avec 1 à 6 atomes de carbone dans la partie alkyle, un benzyloxycarbonyle, un carboxy, un carbamoyle éventuellement substitué par 1 ou 2 restes méthyle ou éthyle, un cyano, un dialcoxyphosphonyle ou un alkylsulfonyle avec 1 à 4 atomes de carbone dans la partie alkyle,

$R^2$ représente l'hydrogène, un alcoxycarbonyle avec 1 à 6 atomes de carbone dans la partie alkyle, un benzyloxycarbonyle, un carbamoyle, un cyano, du chlore, un acétyle, un alkyle avec 1 à 3 atomes de carbone ou un phényle, auquel cas $R^1$ et $R^2$ peuvent également former, conjointement avec l'atome de carbone qu'ils substituent, un cycle oxo-2 tétrahydrofuryle,

$R^3$, $R^4$, $R^5$ et $R^6$ peuvent être identiques ou différents et représentent l'hydrogène, un méthyle, un éthyle, un n- ou un i-propyle et

X représente l'hydrogène, un halogène, de préférence le fluor ou le chlore, ou un nitro, et leurs sels d'addition avec des acides, leurs sels alcalins et alcalino-terreux et leurs hydrates pharmaceutiquement utilisables.

2. Acides quinolone carboxyliques de formule (I) selon la revendication 1, dans laquelle

A représente un alkylène à chaîne droite ou ramifiée avec 1 à 5 atomes de carbone ou >C=CH–,

$R^1$ représente un alcoxycarbonyle avec 1 à 5 atomes de C dans la partie alkyle, un benzyloxycarbonyle, un carboxy, un carbamoyle éventuellement substitué par 1 ou 2 restes méthyle ou éthyle, un cyano, un méthyl- ou éthylsulfonyle,

$R^2$ représente de l'hydrogène, un alcoxycarbonyle avec 1 à 5 atomes de carbone dans la partie alkyle, un benzyloxycarbonyle, un carbamoyle, un cyano, du chlore, de l'acétyle, un alkyle avec 1 à 2 atomes de carbone, un phényle, auquel cas $R^1$ et $R^2$ peuvent également former, conjointement avec l'atome de C qu'ils substituent, un cycle oxo-2 tétrahydrofuryle,

$R^3$, $R^4$, $R^5$ et $R^6$ représentent de l'hydrogène, un méthyle ou un éthyle et

X représente de l'hydrogène, le fluor ou un nitro.

3. Acides quinolone carboxyliques de formule (I) selon la revendication 1, dans laquelle

A représente un alkylène à chaîne droite avec 1 à 5 atomes de carbone ou >C=CH–,

$R^1$ représente un alcoxycarbonyle avec 1 à 4 atomes de C dans la partie alkyle, un benzyloxycarbonyle, un carboxy, un carbamoyle, un cyano, un méthylsulfonyle,

$R^2$ représente de l'hydrogène, un alcoxycarbonyle avec 1 à 3 atomes de C dans la partie alkyle, un cyano, du chlore, un acétyle, un phényle, auquel cas $R^1$ et $R^2$ peuvent également former, conjointement avec l'atome de C qu'ils substituent, un cycle oxo-2 tétrahydrofuryle-3,

$R^3$ représente de l'hydrogéne, un méthyle, un éthyle,

$R^4$ représente de l'hydrogène,

$R^5$ représente de l'hydrogène, un méthyle,

$R^6$ représente de l'hydrogène, et

X représente l'hydrogène, le fluor, le chlore, un nitro.

4. L'acide cyclopropyl-1 fluoro-6-dihydro-1,4-oxo-4 {[oxo-2 (méthoxycarbonyl)-1 propyl-1]-4 pipérazinyl-1}-7 quinoléine-3 carboxylique.

5. L'acide [(chloro-2 cyano-2 éthyl) -4-pipérazinyl-1]-7 cyclopropyl-1 fluoro-6 dihydro-1,4 oxo-4-quinoléine-3 carboxylique.

6. Utilisation des acides quinolone carboxyliques de formule (I)

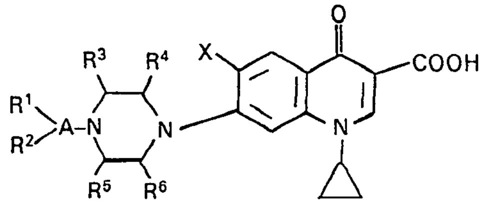

(I)

dans laquelle

A représente un alkylène à chaîne droite ou ramifiée avec 1 à 6 atomes de carbone ou un reste, >C=CH–,

$R^1$ représente un alcoxycarbonyle avec 1 à 6 atomes de carbone dans la partie alkyle, un benzyloxycarbonyle, un carboxy, un carbamoyle éventuellement substitué par 1 ou 2 restes méthyle ou éthyle, un cyano, un dialcoxyphosphonyle ou un alkylsulfonyle avec 1 à 4 atomes de carbone dans la partie alkyle,

$R^2$ représente l'hydrogène, un alcoxycarbonyle avec 1 à 6 atomes de carbone dans la partie alkyle, un benzyloxycarbonyle, un carbamoyle, un cyano, du chlore, un acétyle, un alkyle avec 1 à 3 atomes de carbone ou un phényle, auquel cas $R^1$ et $R^2$ peuvent également former, conjointement avec l'atome de carbone qu'ils substituent, un cycle oxo-2 tétrahydrofuryle,

$R^3$, $R^4$, $R^5$ et $R^6$ peuvent être identiques ou différents et représentent l'hydrogène, un méthyle, un éthyle, un n- ou un i-propyle et

X représente l'hydrogène, un halogène, de préférence le fluor ou le chlore, ou un nitro, et de leurs sels d'addition avec des acides, de leurs sels alcalins et alcalino-terreux et de leurs hydrates pharmaceutiquement utilisables pour la fabrication de médicaments.

7. Procédé de préparation d'acides quinolone carboxylique de formule (I)

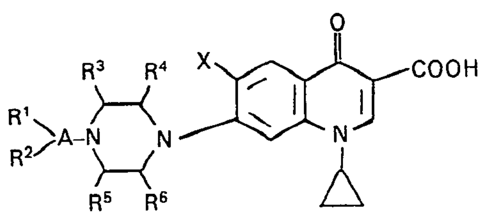

(I)

dans laquelle

A représente un alkylène à chaîne droite ou ramifiée avec 1 à 6 atomes de carbone ou un reste >C=CH–,

$R^1$ représente un alcoxycarbonyle avec 1 à 6 atomes de carbone dans la partie alkyle, un benzyloxycarbonyle, un carboxy, un carbamoyle éventuellement substitué par 1 ou 2 restes méthyle ou éthyle, un cyano, un dialcoxyphosphonyle ou un alkylsulfonyle avec 1 à 4 atomes de carbone dans la partie alkyle,

$R^2$ représente l'hydrogène, un alcoxycarbonyle avec 1 à 6 atomes de carbone dans la partie alkyle, un benzyloxycarbonyle, un carbamoyle, un cyano, du chlore, un acétyle, un alkyle avec 1 à 3 atomes de carbone ou un phényle, auquel cas $R^1$ et $R^2$ peuvent également former, conjointement avec l'atome de carbone qu'ils substituent, un cycle oxo-2 tétrahydrofuryle,

$R^3$, $R^4$, $R^5$ et $R^6$ peuvent être identiques ou différentes et représentent l'hydrogène, un méthyle, un éthyle, un n- ou un i-propyle et

X représente l'hydrogène, un halogène, de préférence le fluor ou le chlore, ou un nitro, et de leurs sels d'addition avec des acides, de leurs sels alcalins et alcalino-terreux et de leurs hydrates pharmaceutiquement utilisables, caractérisé en ce que l'on fait réagir

a) des composés de formule (II)

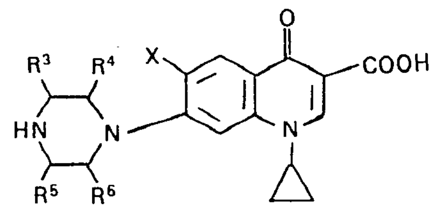

(II)

dans laquelle

X, $R^3$, $R^4$, $R^5$ et $R^6$ possèdent la signification précédente, avec des composés de formule (III)

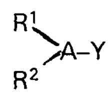

(III)

dans laquelle

$R^1$, $R^2$ et A ont la signification précédente, et

Y représente un halogène, de préférence le chlore, le brome ou l'jode, $CH_3O$–$SO_2$–O, $C_2H_5O$–$SO_2$–O, un méthoxy ou un éthoxy, que l'on fait réagir

b) des composés de formule (II) avec des composés de formule (IV)

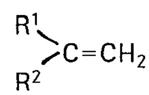

(IV)

dans laquelle

$R^1$ et $R^2$ ont la signification précédente

ou que l'on fait réagir, dans des conditions alcalines

ou acides ou, dans le cas de R'=benzyle, également dans des conditions hydrogénolytiques,

c) des composés de formule (V)

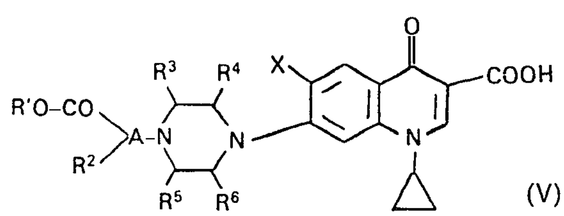

(V)

dans laquelle

$R^2$, $R^3$, $R^4$, $R^5$, $R^6$ et X ont la signification précédente et

R' représente un alkyle avec 1 à 6 atomes de carbone ou un benzyle.

8. Produit pharmaceutique, caractérisé en ce

qu'il contient au moins un acide quinolone carboxylique de formule (I) selon la revendication 1.

9. Acides quinolone carboxyliques de formule (I)

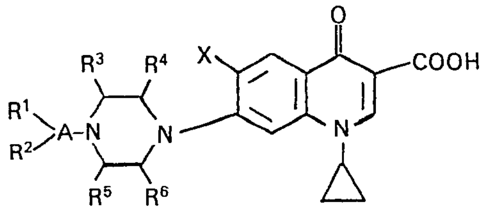

(I)

dans laquelle

A représente un alkylène à chaîne droite ou ramifiée avec 1 à 6 atomes de carbone ou un reste >C=CH–,

$R^1$ représente un alcoxycarbonyle avec 1 à 6 atomes de carbone dans la partie alkyle, benzyloxycarbonyle, un carboxy, un carbamoyle éventuellement substitué par 1 ou 2 restes méthyle ou éthyle, un cyano, un dialcoxyphosphonyle ou un alkyksulfonyle avec 1 à 4 atomes de carbone dans la partie alkyle,

$R^2$ représente l'hydrogène, un alcoxycarbonyle avec 1 à 6 atomes de carbone dans la partie alkyle, un benzyloxycarbonyle, un carbamoyle, un cyano, du chlore, un acétyle, un alkyle avec 1 à 3 atomes de carbone ou un phényle, auquel cas $R^1$ et $R^2$ peuvent également former, conjointement avec l'atome de carbone qu'ils substituent, un cycle oxo-2 tétrahydrofuryle,

$R^3$, $R^4$, $R^5$ et $R^6$ peuvent être identiques ou différents et représentent l'hydrogène, un méthyle, un éthyle, un n- ou un i-propyle et

X représente l'hydrogène, un halogène, de préférence le fluor ou le chlore, ou un nitro

et leurs sels d'addition avec des acides, leurs sels alcalins et alcalino-terreux et leurs hydrates pharmaceutiquement utilisables, destinés à l'emploi dans un procédé pour le traitement thérapeutique du corps humain ou animal.

## Claims

1. Quinolonecarboxylic acids of the formula (I)

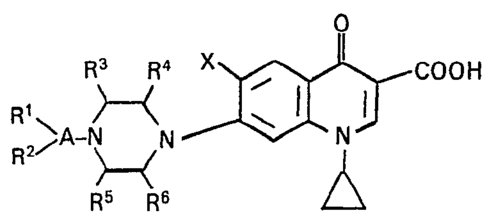

(I)

in which

A represents straight-chain or branched alkylene with 1 to 6 carbon atoms or a radical >C=CH–,

$R^1$ represents alkoxycarbonyl with 1 to 6 carbon atoms in the alkyl part, benzyloxycarbonyl, carboxyl, carbamoyl which is optionally substituted by 1 or 2 methy or ethyl radicals, cyano, dialkoxyphosphonyl or alkylsulophonyl with 1 to 4 carbon atoms in the alkyl part,

$R^2$ represents hydrogen, alkoxycarbonyl with 1 to 6 carbon atoms in the alkyl part, benzyloxycarbonyl, carbamoyl, cyano, chlorine, acetyl, alkyl with 1 to 3 carbon atoms or phenyl, it also being possible for $R^1$ and $R^2$, together with the carbon atom which they substitute, to form a 2-oxotetrahydrofuryl ring and

$R^3$, $R^4$, $R^5$ and $R^6$ can be identical or different and represent hydrogen, methyl, ethyl, n- or i-propyl, and

X represents hydrogen, halogen, preferably fluorine or chlorine, or nitro, and their pharmaceutically usable acid addition, alkali metal and alkaline earth metal salts and hydrates.

2. Quinolonecarboxylic acids of the formula (I) in Claim 1, in which

A denotes straight-chain or branched alkylene with 1 to 5 C atomes or >C=CH–,

$R^1$ denotes, alkoxycarbonyl with 1 to 5 C atomes in the alkyl part, benzyloxycarbonyl, carboxyl, carbamoyl which is optionally substituted by 1 or 2 methyl or ethyl radicals, cyano, methylsulphonyl or ethylsulphonyl,

$R^2$ denotes hydrogen, alkoxycarbonyl with 1 to 5 C atoms in the alkyl part, benzyloxycarbonyl, carbamoyl, cyano, chlorine, acetyl, alkyl with 1 to 2 carbon atoms or phenyl, it also being possible for $R^1$ and $R^2$, together with the C atom which they substitute, to form a 2-oxo-tetrahydrofuryl ring,

$R^3$, $R^4$, $R^5$ and $R^6$ denote hydrogen, methyl or ethyl and

X denotes hydrogen, fluorine or nitro.

3. Quinolonecarboxylic acids of the formula (I) in Claim 1, in which

A denotes straight-chain alkylene with 1 to 5 C atoms or >C=CH–,

$R^1$ denotes alkoxycarbonyl with 1 to 4 C atoms in the alkyl part, benzyloxycarbonyl, carboxyl, carbamoyl, cyano or methylsulphonyl,

$R^2$ denotes hydrogen, alkoxycarbonyl with 1 to 3 C atoms in the alkyl part, cyano, chlorine, acetyl or phenyl, it also being possible for $R^1$ and $R^2$, together with the C atom which they substitute, to form a 2-oxo-tetrahydro-3-furyl ring,

$R^3$ denotes hydrogen, methyl or ethyl,

$R^4$ denotes hydrogen,

$R^5$ denotes hydrogen or methyl,

$R^6$ denotes hydrogen and

X denotes hydrogen, fluorine, chlorine or nitro.

4. 1-Cyclopropyl- 6- fluoro- 1,4- dihydro- 4-oxo- 7-{4- [2-oxo- 1-(methoxycarbonyl)-1-propyl]-1- piperazinyl} -3-quinolinecarboxylic acid.

5. 7-[4-(2-Chloro-2-cyano-ethyl) -1-piperazinyl] -1-cyclopropyl- 6-fluoro- 1,4- dihydro-4-oxo- 3-quinolinecarboxylic acid.

6. Use of quinolonecarboxylic acids of the formula (I)

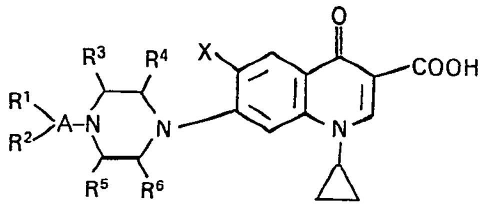

(I)

in which

A represents straight-chain or branched alkylene with 1 to 6 carbon atoms or a radical >C=CH–,

$R^1$ represents alkoxycarbonyl with 1 to 6 carbon atoms in the alkyl part, benzyloxycarbonyl, carboxyl, carbamoyl which is optinally substituted by 1 or 2 methyl or ethyl radicals, cyano, dialkoxyphosphonyl or alkylsulphonyl with 1 to 4 carbon atoms in the alkyl part,

$R^2$ represents hydrogen, alkoxycarbonyl with 1 to 6 carbon atoms in the alkyl part, benzyloxycarbonyl, carbamoyl, cyano, chlorine, acetyl, alkyl with 1 to 3 carbon atoms or phenyl, it also being possible for $R^1$ and $R^2$, together with the carbon atom which they substitute, to form a 2-oxotetrahydrofuryl ring, and

$R^3$, $R^4$, $R^5$ and $R^6$ can be identical or different and represent hydrogen, methyl, ethyl, n- or i-propyl, and

X represents hydrogen, halogen, preferably fluorine or chlorine, or nitro, and their pharmaceutically usable acid addition, alkali metal and alkaline earth metal salts and hydrates for the preparation of medicaments.

7. Process for the preparation of quinolonecarboxylic acids of the formula (I)

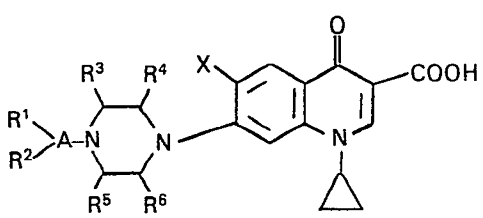

(I)

in which

A represents straight-chain or branched alkylene with 1 to 6 carbon atoms or a radical >C=CH–,

$R^1$ represents alkoxycarbonyl with 1 to 6 carbon atoms in the alkyl part, benzyloxycarbonyl, carboxyl, carbamoyl which is optionally substituted by 1 or 2 methyl or ethyl radicals, cyano, dialkoxyphosphonyl or alkylsulphonyl with 1 to 4 carbon atoms in the alkyl part,

$R^2$ represents hydrogen, alkoxycarbonyl with 1 to 6 carbon atoms in the alkyl part, benzyloxycarbonyl, carbamoyl, cyano, chlorine, acetyl, alkyl with 1 to 3 carbon atoms or phenyl, it also being possible for $R^1$ and $R^2$, together with the carbon atom which they substitute, to form a 2-oxotetrahydrofuryl ring and

$R^3$, $R^4$, $R^5$ and $R^5$ can be identical or different and represent hydrogen, methyl, ethyl, n- or i-propyl, and

X represents hydrogen, halogen, preferably fluorine or chlorine, or nitro, and their pharmaceutically usable acid addition, alkali metal and alkaline earth metal salts and hydrates, characterised in that either

a) compounds of the formula (II)

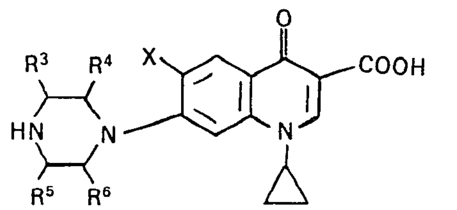

(II)

in which

X, $R^3$, $R^4$, $R^5$ and $R^6$ have the abovementioned meaning, are reacted with compounds of the formula (III)

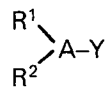

(III)

in which

$R^1$, $R^2$ and A have the abovementioned meaning and

Y represents halogen, preferably chlorine, bromine or iodine, $CH_3O{-}SO_2{-}O$, $C_2H_5O{-}SO_2{-}O$, methoxy or ethoxy, or in that

b) compounds of the formula (II) are reacted with compounds of the formula (IV)

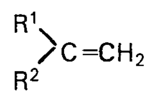

(IV)

in which

$R^1$ and $R^2$ have the abovementioned meaning, or in that

c) compounds of the formula (V)

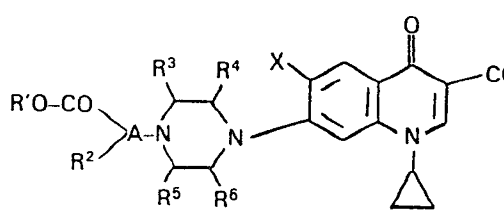

(V)

in which

$R^2$, $R^3$, $R^4$, $R^5$, $R^6$ and X have the abovementioned meaning and

R′ represents alkyl with 1 to 6 carbon atoms or benzyl, are reacted under alkaline or acid conditions or, if R′ = benzyl, also under hydrogenolytic conditions.

8. Medicaments, characterised in that they contain at least one quinolonecarboxylic acid of the formula (I) in Claim 1.

9. Quinolonecarboxylic acids of the formula (I)

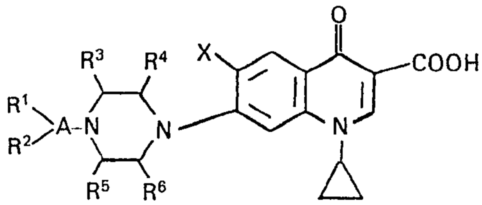

(I)

in which

A represents straight-chain or branched alkylene with 1 to 6 carbon atoms or a radical >C=CH–,

$R^1$ represents alkoxycarbonyl with 1 to 6 carbon atoms in the alkyl part, benzyloxycarbonyl, carboxyl, carbamoyl which is optionally substituted by 1 or 2 methyl or ethyl radicals, cyano, dialkoxyphosphonyl or alkylsulphonyl with 1 to 4 carbon atoms in the alkyl part,

$R^2$ represents hydrogen, alkoxycarbonyl with 1 to 6 carbon atoms in the alkyl part, benzyloxycarbonyl, carbamoyl, cyano, chlorine, acetyl, alkyl with 1 to 3 carbon atoms or phenyl, it also being possible for $R^1$ and $R^2$, together with the carbon atom which they substitute, to form a 2-oxotetrahydrofuryl ring and

$R^3$, $R^4$, $R^5$ and $R^6$ can be identical or different and represent hydrogen, methyl, ethyl, n- or i-propyl and

X represents hydrogen, halogen, preferably fluorine or chlorine, or nitro, and their pharmaceutically usable acid addition, alkali metal and alkaline earth metal salts and hydrates, for use in a procedure for the therapeutic treatment of the human or animal body.